(19) Europäisches Patentamt
European Patent Office
Office européen des brevets

(11) Veröffentlichungsnummer : **0 043 788**
**B1**

(12) # EUROPÄISCHE PATENTSCHRIFT

(45) Veröffentlichungstag der Patentschrift : **30.10.85**

(21) Anmeldenummer : **81730061.9**

(22) Anmeldetag : **30.06.81**

(51) Int. Cl.[4] : **C 07 D233/84**, C 07 D233/64, C 07 D401/04, C 07 D401/12, C 07 D403/12, C 07 D405/04, C 07 D409/04, C 07 D409/12, C 07 D417/12, A 61 K 31/415

(54) **Neue Imidazol-Derivate, Verfahren zu ihrer Herstellung und diese enthaltende pharmazeutische Präparate.**

(30) Priorität : **03.07.80 DE 3025484**

(43) Veröffentlichungstag der Anmeldung : **13.01.82 Patentblatt 82/02**

(45) Bekanntmachung des Hinweises auf die Patenterteilung : **30.10.85 Patentblatt 85/44**

(84) Benannte Vertragsstaaten :
**AT BE CH DE FR GB IT LI LU NL SE**

(56) Entgegenhaltungen :
**EP-A- 0 004 648**
**EP-A- 0 005 219**
**EP-A- 0 005 545**
**EP-A- 0 013 732**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Niedballa, Ulrich, Dr.**
**Gosslerstrasse 28a**
**D-1000 Berlin 33 (DE)**
Erfinder : **Böttcher, Irmgard, Dr.**
**Rodelbahnpfad 5**
**D-1000 Berlin 28 (DE)**

**EP 0 043 788 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Aus EP-A-0 005 545 bzw. DE-A-2 823 197 sind antiinflammatorisch und antiallergisch wirkende Imidazolderivate entsprechend Formel I gemäss Anspruch 1 bekannt, wobei jedoch Z abweichend einen gesättigten oder ungesättigten $C_{2-6}$-Kohlenwasserstoffrest darstellt.

Die Erfindung betrifft neue Imidazol-Derivate der allgemeinen Formel I gemäß Anspruch 1, ein Verfahren zu ihrer Herstellung und pharmazeutische Präparate, die diese Imidazol-Derivate als Wirkstoffe enthalten.

Erfindungsgemäß bedeuten die Reste $R_2$ und $R_3$ der Imidazolderivate jeweils einen gegebenenfalls durch Halogen-atome, 1 bis 4 Kohlenstoffatome enthaltende Alkylreste oder 1 bis 4 Kohlenstoffatome enthaltende Alkoxyreste substituierten Phenylrest. Durch Halogenatome substituierte Phenylreste $R_2$ und $R_3$ sind beispielsweise Mono- oder Difluorphenylreste oder Mono- oder Dichlorphenylreste und insbesondere p-Fluorphenylreste oder p-Chlorphenylreste. Alkylsubstituierte Phenylreste sind solche, deren Alkylgruppen beispielsweise Methyl-, Ethyl-, Propyl- oder Isopropylgruppen sind. Durch Alkoxygruppen substituierte Phenylreste sind solche, deren Alkoxygruppen beispielsweise Methoxy-, Ethoxy-, Propyloxy- oder Isopropyloxygruppen sind.

Die Phenylreste $R_2$ und $R_3$ können einfach oder mehrfach substituiert sein mit gleichen oder verschiedenen Substituenten. Vorzugsweise sind sie einfach substituiert. Besonders bevorzugt sind die in Anspruch 2 und 3 genannten Reste $R_2$ und $R_3$ und insbesondere der p-Methoxyphenylrest.

Der Rest $R_1$ der Imidazol-Derivate der allgemeinen Formel I ist erfindungsgemäß ein Wasserstoffatom oder ein gegebenenfalls durch Methoxygruppen substituierter Alkylrest mit 1 bis 6 Kohlenstoffatomen, wobei Wasserstoff bevorzugt ist.

Erfindungsgemäß ist der Substituent Z der Imidazol-Derivate der allgemeinen Formel I gemäß Anspruch 1 ein gegebenenfalls durch Halogenatome, 1 bis 4 Kohlenstoffatome enthaltende Alkylgruppen oder 1 bis 4 Kohlenstoffatome enthaltende Alkoxygruppen substituierter Phenylrest, Pyridylrest, N-Oxipyridylrest, Pyrimidinylrest, Thiazolylrest oder Thienylrest. Bevorzugte Substituenten Z sind der 2-Thienylrest, solche Reste, in denen Z die gleiche Bedeutung besitzt wie die Substituenten $R_2$ und $R_3$, insbesondere der 2-Fluorphenylrest und der 4-Fluor-phenylrest der 4-Pyridylrest sowie.

Physiologisch unbedenkliche Salze der Imidazol-Derivate der allgemeinen Formel I sind beispielsweise Salze des Chlorwasserstoffs, Bromwasserstoffs oder Jodwasserstoffs, der Schwefelsäure, Phosphorsäure oder Salze organischer Säuren wie der Ameisensäure, Essigsäure, Bernsteinsäure, Maleinsäure, Weinsäure oder der Zitronensäure.

Die Imidazol-Derivate der allgemeinen Formel I gemäß Anspruch 1 zeichnen sich durch eine ausgeprägte antiinflammatorische und antiallergische Wirksamkeit aus.

Darüberhinaus zeichnen sich die Imidazol-Derivate der allgemeinen Formel dadurch aus, daß sie eine sehr günstige Dissoziation zwischen erwünschter pharmakologischer Wirksamkeit und unerwünschten — insbesondere ulcerogenen — Nebenwirkungen besitzen.

Die antiinflammatorische Wirksamkeit der erfindungsgemäßen Substanzen kann mit Hilfe des bekannten Adjuvans-Arthritis-Testes ermittelt werden, der wie folgt durchgeführt wird :

Es werden weibliche und männliche Ratten des Stammes Lewis (LEW) in der Gewichtsspanne zwischen 110-190 g verwendet. Die Tiere erhalten Trinkwasser und Altromin-Preßfutter *ad libitum.*

Für jede Dosisgruppe werden 10 Ratten eingesetzt.

Mycobacterium butyricum der Firma Difko, Detroit wird als Reizmittel verwandt. Eine Suspension von 0,5 mg Mycobacterium butyricum in 0,1 ml dünnflüssigen Paraffin (DAB 7) wird in die rechte Hinterpfote subplantar injiziert.

Die Testsubstanzen werden vom 11. Versuchstag an täglich über 4 Tage oral gegeben. Die Substanzen werden als klare wässrige Lösung oder als Kristallsuspension unter Zusatz von Myrj 53 (85 mg%) in isotonischer Natriumchlorid-Lösung verabreicht.

Versuchsansatz :

Die Ratten werden in bezug auf ihr Körpergewicht möglichst gleichmäßig in verschiedene Gruppen eingeteilt. Nach plethysmographischer Volumenmessung der rechten Hinterpfote wird in diese subplantar 0,1 ml Adjuvans injiziert.

Die rechten Hinterpfoten werden vom 14. Versuchstag bis zu Versuchsende gemessen. Die Versuchsdauer beträgt 3 Wochen.

Bestimmt wird die Dosis, bei der eine 40 %ige Abnahme des Pfotenvolumens im Vergleich zum unbehandelten Tier erzielt wird ($ED_{40}$ in mg/kg Körpergewicht).

Eine häufige Komplikation bei der Therapie mit nichtsteroidalen Entzündungshemmern stellt das Auftreten von Magenulcerationen dar. Diese Nebenwirkung kann im Tierversuch nachgewiesen werden, wobei bei vorgegebener Dosis die Anzahl der beobachteten Läsionen und deren Gesamtfläche ermittelt wird. Der Ulkus-Test wird wie folgt durchgeführt.

Es werden männliche Wistar-Ratten (SPF) verwandt. Die Tiere liegen in einer Gewichtsspanne von $130 \pm 10$ g. 16 Stunden vor Versuchsbeginn werden die Tiere vom Futter abgesetzt ; sie erhalten Wasser *ad libitum.*

Pro Dosis werden 5 Tiere eingesetzt. Die Substanzen werden einmal oral, in Natriumchlorid gelöst oder als Kristallsuspension unter Zusatz von 85 mg% Myrj 53 appliziert.

3 Stunden nach Substanzapplikation injiziert man 1 ml einer 3 %igen Lösung der Farbstoffs Diphenylreinblau intravenös und tötet das Tier. Der Magen wird reseziert und mikroskopisch auf Anzahl von Ulcera, die durch Farbstoffanreicherungen hervortreten, untersucht.

Es wird die Dosis ermittelt, bei der mehr als drei Ulcera pro Tier ermittelt wurden (= « Ulcerogene Dosis » in mg/kg Körpergewicht).

Die nachfolgende Tabelle zeigt die in diesen Testen erhaltenen Ergebnisse der erfindungsgemäßen Verbindungen D bis G im Vergleich zum vorbekannten Indomethacin (Substanz A) zu Phenylbutazon (Substanz B) und zu der aus der deutschen Offenlegungsschrift 2 823 197 vorbekannten strukturanalogen Verbindung C.

| Nr. | Substanz | Adjuvans-Arthritis Test $ED_{40}$ mg/kg | "Ulcerogene Dosis" in mg/kg |
|---|---|---|---|
| A | Indomethacin | 8 | 3 |
| B | Phenylbutazon | 50 | 80 |
| C | 4,5-Bis-(4-methoxyphenyl)-2-(2-hydroxy-ethylsulfinyl)-imidazol (Deutsche Offenlegunsschrift 28 23 197) | 75 | größer 400 |
| D | 4,5-Bis-(4-methoxyphenyl)-2-(4-trifluormethylphenylthio)-imidazol | 30 | größer 400 |
| E | 4,5-Bis-(4-methoxyphenyl)-2-phenylthio-imidazol | 28 | größer 400 |
| F | 4,5-Bis-(4-methoxyphenyl)-2-phenylsulfonyl-imidazol | 25 | größer 400. |
| G | 4,5-Bis-(4-methoxyphenyl)-2-(2-thienylthio)-imidazol | 20 | größer 400 |

Somit eignen sich die neuen Verbindungen in Kombination mit den in der galenischen Pharmazie üblichen Trägermitteln zur Behandlung zum Beispiel von akuter oder chronischer Polyarthritis, Neurodermitis, Asthma bronchiale, Heufieber u. a.

Bemerkenswert ist, daß sich die Imidazol-Derivate der allgemeinen Formel I gemäß Anspruch 1 darüberhinaus auch zur Behandlung von Migräne und von Dysmenorrhoe eignen.

Überraschenderweise gibt es unter den erfindungsgemäßen Verbindungen auch solche, die zusätzlich zur antiinflammatorischen Wirksamkeit noch eine ausgeprägte antiulcerogene, tumorhemmende Wirksamkeit besitzen.

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Inhalationsmitteln usw. überführt.

Für die orale Anwendung eignen sich insbesondere Tabletten, Dragees und Kapseln, welche beispielsweise 1 bis 250 mg Wirkstoff und 50 mg bis 2 g mindestens eines pharmakologisch unwirksamen Trägers, wie zum Beispiel Laktose, Amylose, Talkum, Gelatine, Magnesiumstearat und ähnliches, sowie die üblichen Zusätze enthalten.

Die neuen Imidazol-Derivate der allgemeinen Formel I gemäß Anspruch 1 können nach an sich bekannten Verfahren hergestellt werden. Ein geeignetes Herstellungsverfahren ist beispielsweise die im Anspruch 18 beschriebene Synthese.

Die im Anspruch 18 aufgeführten Gegebenenfallsmaßnahmen sind vorzugsweise die Oxidation von Thioverbindungen der allgemeinen Formel I zu den entsprechenden Sulfinylverbindungen oder Sulfonylverbindungen und/oder die Oxidation von Imidazol-Derivaten der allgemeinen Formel I mit Z in der

Bedeutung eines Pyridylrestes zu solchen mit Z in der Bedeutung eines N-Oxipyridylrestes, die Hydrolyse von Verbindungen der allgemeinen Formel I in welchen $R_1$ einen von Wasserstoff verschiedenen Substituenten darstellt, und die Alkylierung von Verbindungen der allgemeinen Formel I mit $R_1$ in der Bedeutung von Wasserstoff.

Diese Synthese kann unter an sich bekannten Bedingungen durchgeführt werden (Houben-Weyl : Methoden der Org. Chemie IX, Georg Thieme Verlag, Stuttgart (1955), Seite 166 ff und 229 ff ; Chemistry Letters 1979, 939 ; Arch. Chim. Acad. Sci Hung. *98*, 1978, 479 ; Org. Synth. *51*, 1971, 128 ; J. Amer. Chem. Soc. *100*, 1978, 2510 und 3918 ; Chem. Rev. *4*, 1978, 364 ; J. Org. Chem. *43*, 1979, 1379 ; Tetrahedron Letters 1975, 921 ; Deutsche Offenlegungsschrift 26 35 876 ; Rec. Trav. Chim. Pays-Bas *98*, 1979, 371 ; J. Med. Chem. *16*, 1973, 1161 und Bull. Soc. Chim. France 1977, 271, Dokl. Acad. Nauc SSSR Ser. Khim *249*, 1979, 867).

Nach erfolgter Synthese können die racemischen Imidazol-Derivate der allgemeinen Formel I gemäß Anspruch 1 in an sich bekannter Weise in ihre optischen Antipoden gespalten werden, beispielsweise indem man diese durch Säulenchromatographie an optisch aktiven Trägern (z. B. Sephadex[(R)]) chromatographiert. Führt man die Synthese nach der Verfahrensvariante b) des Anspruchs 18 durch und verwendet als Ausgangssubstanzen optisch aktive Verbindungen der allgemeinen Formeln VII oder VIII, so erhält man unmittelbar die entsprechenden optisch aktiven Imidazol-Derivate der allgemeinen Formel I gemäß Anspruch 1.

Die Ausgangsverbindungen für das erfindungsgemäße Verfahren gemäß Anspruch 18 sind bekannt oder können in an sich bekannter Weise hergestellt werden (Zhur. Obschei Khim *31*, 1961, 1093 ; Synthesis 1976, 733 ; Südafrikanisches Patent 64/4808 ; J. Chem. Soc. 1963, 2135 ; Rec. Trav. Chim. Pays-Bas *98*, 1979, 371 ; Deutsche Offenlegungsschrift 26 35 876 ; Chem. Pharm. Bull *27*, 1979, 899 ; Houben-Weyl, Methoden der Org. Chemie IX, Georg Thieme Verlag Stuttgart, 1955, Seite 117 ff und Seite 552 ff ; Org. Synth. *51*, 1971, 128 ; Bull. Chem. Soc. Japan, *52*, 1979, 2635 ; J. Med. Chem. *22*, 1979, 1483 ; Synthesis 1980, 32 ; Synthesis 1979, 181 ; J. prakt. Chem. *321*, 1979, 495 ; J. Org. Chem., 30, 1965, 633 ; Rec. Trav. Chim. Pays-Bas *73*, 1954, 129 und J. Amer. Chem. Soc. *100*, 1978, 2510).

Die nachfolgenden Ausführungsbeispiele dienen zur Erläuterung des erfindungsgemäßen Verfahrens :

## Beispiel 1

In 50 ml absolutem Dimethylformamid werden 3,12 g 4,5-Bis-(4-methoxyphenyl)-2-mercaptoimidazol gelöst und mit 0,3 g Natriumhydrid (80 %ig in Weißöl) versetzt. Man läßt 30 Minuten nachrühren, tropft dann 2,31 g 2-Jodthiophen in 25 ml Dimethylformamid zu, versetzt mit einer Spatelspitze Kupferpulver und läßt 12 Stunden unter Argon bei 100 °C rühren. Dann gießt man die Lösung in 300 ml Eiswasser, extrahiert das Produkt mit Essigester, trocknet die organische Lösung über Natriumsulfat und engt sie im Vakuum zur Trockne ein. Der Rückstand wird an 200 g Kieselgel chromatographiert. Mit Aceton/Hexan 2 : 3 eluiert man 3,06 g 4,5-Bis-(4-methoxyphenyl)-2-(2-thienylthio)-imidazol, das nach Umkristallisation aus Diisopropylether einen Schmelzpunkt von 156 °C zeigt.

## Beispiel 2

In 50 ml absolutem Dimethylformamid werden 3,12 g 4,5-Bis-(4-methoxyphenyl)-2-mercaptoimidazol gelöst und mit 0,3 g Natriumhydrid (80 %ig in Weißöl) versetzt. Man läßt 30 Minuten nachrühren, tropft dann 1,74 g 2-Brompyridin in 25 ml Dimethylformamid zu, und läßt 30 Stunden unter Argon bei Raumtemperatur rühren. Dann gießt man die Lösung in 300 ml Eiswasser, extrahiert das Produkt mit Essigester, trocknet die organische Lösung über Natriumsulfat und engt sie im Vakuum zur Trockne ein. Der Rückstand wird aus Methylenchlorid/Ether umkristallisiert und man erhält 2,94 g 4,5-Bis-(4-methoxyphenyl)-2-(2-pyridylthio)-imidazol, vom Schmelzpunkt 94 °C.

## Beispiel 3

In 50 ml absolutem Dimethylformamid werden 3,12 g 4,5-Bis-(4-methoxyphenyl)-2-mercaptoimidazol gelöst und mit 0,3 g Natriumhydrid (80 %ig in Weißöl) versetzt. Man läßt 30 Minuten nachrühren, tropft dann 1,81 g 2-Bromthiazol in 25 ml Dimethylformamid zu, versetzt mit einer Spatelspitze Kupferpulver und läßt 6 Stunden unter Argon bei 80 °C rühren. Dann gießt man die Lösung in 300 ml Eiswasser, extrahiert das Produkt mit Essigester, trocknet die organische Lösung über Natriumsulfat und engt sie im Vakuum zur Trockne ein. Der Rückstand wird an 200 g Kieselgel chromatographiert. Mit Aceton/Hexan eluiert man 3,14 g 4,5-Bis-(4-methoxyphenyl)-2-(2-thiazolylthio)-imidazol, das nach Umkristallisation aus Diisopropylether einen Schmelzpunkt von 145 °C zeigt.

## Beispiel 4

In 50 ml absolutem Dimethylformamid werden 3,12 g 4,5-Bis-(4-methoxyphenyl)-2-mercaptoimidazol gelöst und mit 0,3 g Natriumhydrid (80 %ig in Weißöl) versetzt. Man läßt 30 Minuten nachrühren, tropft

dann 1,26 g 2-Chlorpyrimidin in 25 ml Dimethylformamid zu, versetzt mit einer Spatelspitze Kupferpulver und läßt eine Woche unter Argon bei Raumtemperatur rühren. Dann gießt man die Lösung in 300 ml Eiswasser, extrahiert das Produkt mit Essigester, trocknet die organische Lösung über Natriumsulfat und engt sie im Vakuum zur Trockne ein. Der Rückstand wird aus Eisessig/Ether umkristallisiert und man erhält 2,87 g 4,5-Bis-(4-methoxyphenyl)-2-(2-pyrimidinylthio)-imidazol, vom Schmelzpunkt 86 °C.

Beispiel 5

a) Zu einer Lösung von 7,94 g 4,5-Diphenyl-2-mercaptoimidazol und 0,45 g Natriumhydrid (80 %ig in Weißöl) in 150 ml Dimethylformamid wird unter Rühren und Abdeckung mit Argon eine Lösung von 7,45 g 4-Chlor-3-nitro-trifluormethylbenzol in 40 ml Dimethylformamid getropft. Man läßt 20 Minuten nachrühren, engt im Vakuum ein und verteilt den Ruckstand zwischen Wasser und Essigester. Die organische Lösung wird über Natriumsulfat getrocknet und im Vakuum zur Trockne eingeengt. Der Rückstand wird aus Ether kristallisiert. Man erhält so 11,87 g 4,5-Diphenyl-2-(2-nitro-4-trifluor-methyl-phenylthio)-imidazol vom Schmelzpunkt 234-236 °C.

b) Zu einer Lösung von 4,41 g 4,5-Diphenyl-2-(2-nitro-4-trifluormethyl-phenylthio)-imidazol in einem Gemisch aus 100 ml Dioxan, 48 ml Wasser und 2 ml konz. Ammoniak gibt man bei 60° unter intensivem Rühren 4,2 g Natriumdithionit (ca. 87 %ig). Die Farbe der Lösung schlägt von rotbraun nach blaßgelb um. Man gießt in 700 ml Eiswasser, extrahiert mit Essigester, trocknet die organische Lösung über Natriumsulfat und engt sie im Vakuum zur Trockne ein. Der Rückstand wird in Ethanol gelöst und mit 10 ml etherischer Salzsäure versetzt. Beim Einengen beginnt das Produkt zu kristallisieren. Nach Umkristallisation aus Ethanol erhählt man 3,89 g 4,5-Diphenyl-2-(2-amino-4-trifluormethylphenylthio)-imidazol Hydrochlorid vom Zersetzungspunkt 177-179 °C.

c) In 100 ml methanolischer Salzsäure werden 2,24 g 4,5-Diphenyl-2-(2-amino-4-trifluormethyl-phenylthio)-imidazol Hydrochlorid gelöst und bei 0 °C mit 0,586 g Isoamylnitrit versetzt. Man läßt 30 Minuten rühren, erwärmt dann 2 Stunden zum Sieden und dampft die Lösung im Vakuum zur Trockne ein. Der Rückstand wird zwischen Essigester und Natriumbikarbonatlösung verteilt. Die organische Lösung wird über Natriumsulfat getrocknet und im Vakuum zur Trockne eingeengt. Der Rückstand wird aus Ether/Chloroform kristallisiert, und man erhält so 1,61 g 4,5-Diphenyl-2-(4-trifluormethyl-phenylthio)-imidazol vom Schmelzpunkt 227-229 °C.

Beispiel 6

a) Zu einer Lösung von 6,25 g 4,5-Bis-(4-methoxyphenyl)-2-mercaptoimidazol und 0,6 g Natriumhydrid (80 %ig in Weißöl) in 200 ml Dimethylformamid wird unter Rühren und Abdeckung mit Argon eine Lösung von 4,52 g 4-Chlor-3-nitro-trifluormethylbenzol in 25 ml Dimethylformamid getropft. Man läßt 20 Minuten nachrühren, engt im Vakuum ein und verteilt den Rückstand zwischen Wasser und Essigester. Die organische Lösung wird über Natriumsulfat getrocknet und im Vakuum zur Trockne eingeengt. Der Rückstand wird aus Hexan/Aceton kristallisiert. Man erhält so 9,44 g 4,5-Bis-(4-methoxyphenyl)-2-(2-nitro-4-trifluormethyl-phenylthio)-imidazol in gelben Nadeln vom Schmelzpunkt 264-266 °C.

b) Zu einer Lösung von 10,3 g 4,5-Bis-(4-methoxyphenyl)-2-(2-nitro-4-trifluormethyl-phenylthio)-imidazol in einem Gemisch aus 300 ml Dioxan, 95 ml Wasser und 5 ml konz. Ammoniak gibt man bei 60 °C unter intensivem Rühren 8,5 g Natriumdithionit (ca. 87 %ig). Die Farbe der Lösung schlägt von rotbraun nach blaßgelb um. Man gießt in 1 l Eiswasser, extrahiert mit Essigester, trocknet die organische Lösung über Natriumsulfat engt sie im Vakuum zur Trockne ein. Der Rückstand wird in Ethanol gelöst und mit 10 ml etherischer Salzsäure versetzt. Beim Einengen beginnt das Produkt zu kristallisieren. Nach Umkristallisation aus Ethanol erhält man 8,28 g 4,5-Bis-(4-methoxyphenyl)-2-(2-amino-4-trifluormethyl-phenylthio)-imidazol Hydrochlorid vom Zersetzungspunkt 200-202 °C.

c) In einem Gemisch aus 70 ml absolutem Ethanol, 30 ml absolutem Dioxan und 365 mg Salzsäure werden 2,61 g 4,5-Bis-(4-methoxyphenyl)-2-(2-amino-4-trifluormethyl-phenylthio)-imidazol Hydrochlorid suspendiert und bei 0 °C mit 0,586 g Isoamylnitrit versetzt. Man läßt 30 Minuten rühren, erwärmt dann 2 Stunden zum Sieden und dampft die Lösung im Vakuum zur Trockne ein. Der Rückstand wird zwischen Essigester und Natriumbikarbonatlösung verteilt. Die organische Lösung wird über Natriumsulfat getrocknet und im Vakuum zur Trockne eingeengt. Der Rückstand wird aus Ether/Methylenchlorid kristallisiert, und man erhält so 2,01 g 4,5-Bis-(4-methoxyphenyl)-2-(4-trifluormethyl-phenylthio)-imidazol vom Schmelzpunkt 184-186 °C.

Beispiel 7

1,5 g Anilin werden in 2,0 g konzentrierter Schwefelsäure und 30 ml Wasser gelöst und nach Zugabe

von 10 g Eis bei 0° mit einer Lösung von 1,2 g Natriumnitrit in 10 ml Wasser diazotiert. Die Diazoniumsalzlösung wird dann mit Natriumacetat abgestumpft und bei 5 °C unter intensivem Rühren in eine Lösung von 5,02 g 4,5-Bis-(4-methoxyphenyl)-2-mercaptoimidazol in 200 ml Dimethylformamid, in der 10 ml Wasser, 1,93 g Natronlauge sowie 1,15 g Kupferpulver enthalten sind, gegeben. Man läßt 20 Minuten nachrühren, gießt in 600 ml Wasser, nimmt das ausgefallene Öl in Methylenchlorid auf, wäscht die organische Lösung mit Wasser, trocknet über Natriumsulfat und engt sie im Vakuum zur Trockne ein. Der Rückstand wird an 200 g Kieselgel chromatographiert. Mit Aceton/Hexan 2 : 3 eluiert man 2,47 g 4,5-Bis-(4-methoxyphenyl)-2-(phenylthio)-imidazol, das nach Umkristallisation aus Hexan/Essigester einen Schmelzpunkt von 177-179 °C zeigt.

Beispiel 8

Zu einer Lösung von 6,25 g 4,5-Bis-(4-methoxyphenyl)-2-mercaptoimidazol in einer Mischung von 240 ml Dimethylformamid, 10 ml 2 N-Natronlauge und 1,45 g Kupferpulver wird bei 0 °C eine Diazoniumsalzlösung getropft, die aus 2,5 g 2-Fluoranilin, 1,58 g Natriumnitrit und 10 ml 6 N Salzsäure bereitet wurde. Die Lösung färbt sich unter Stickstoffentwicklung rotbraun. Man läßt 3 Stunden nachrühren, engt die Lösung im Vakuum ein und verteilt den Rückstand zwischen Wasser und Essigester. Die organische Lösung wird über Natriumsulfat getrocknet und im Vakuum zur Trockne eingeengt. Der Rückstand wird der Säulenchromatographie an 500 g Kieselgel unterworfen. Mit Aceton/Hexan 2 : 3 eluiert man 2,71 g 4,5-Bis)-(4-methoxyphenyl)-2-(2-fluorphenylthio)-imidazol, das nach Umkristallisation aus heißem Diisopropylether einen Schmelzpunkt von 195 °C zeigt.

Beispiel 9

Zu einer Lösung von 6,25 g 4,5-Bis-(4-methoxyphenyl)-2-mercaptoimidazol in einer Mischung von 240 ml Dimethylformamid, 10 ml 2 N Natronlauge und 1,45 g Kupferpulver wird bei 0 °C eine Diazoniumsalzlösung getropft, die aus 2,5 g 4-Fluoranilin, 1,58 g Natriumnitrit und 10 ml 6 N Salzsäure bereitet wurde. Die Lösung färbt sich unter Stickstoffentwicklung rotbraun. Man läßt 3 Stunden nachrühren, engt die Lösung im Vakuum ein und verteilt den Rückstand zwischen Wasser und Essigester. Die organische Lösung wird über Natriumsulfat getrocknet und im Vakuum zur Trockne eingeengt. Der Rückstand wird der Säulenchromatographie an 500 g Kieselgel unterworfen. Mit Aceton/Hexan 2 : 3 eluiert man 3,09 g 4,5-Bis-(4-methoxyphenyl)-2-(4-fluorphenylthio)-imidazol, das nach Umkristallisation aus heißem Diisopropylether/Methylenchlorid einen Schmelzpunkt von 106 °C zeigt.

Beispiel 10

Zu einer Lösung von 6,25 g 4,5-Bis-(4-methoxyphenyl)-2-mercaptoimidazol in einer Mischung von 240 ml Dimethylformamid, 10 ml 2 N Natronlauge und 1,45 g Kupferpulver wird bei 0 °C eine Diazoniumsalzlösung getropft, die aus 2,58 g 2,4-difluoranilin, 1,58 g Natriumnitrit und 10 ml 6 N Salzsäure bereitet wurde. Die Lösung färbt sich unter Stickstoffentwicklung rotbraun. Man läßt 3 Stunden nachrühren, engt die Lösung im Vakuum ein und verteilt den Rückstand zwischen Wasser und Essigester. Die organische Lösung wird über Natriumsulfat getrocknet und im Vakuum zur Trockne eingeengt. Der Rückstand wird der Säulenchromatographie an 500 g neutralem Aluminiumoxid (Aktivitätsstufe II) unterworfen. Mit Aceton/Hexan 2 : 3 eluiert man 3,12 g 4,5-Bis-(4-methoxyphenyl)-2-(2,4-difluorphenylthio)-imidazol, das nach Kristallisation aus Hexan/Ether einen Schmelzpunkt von 174-176 °C und nach nochmaliger Kirstallisation aus Ethanol/Ether einem Schmelpunkt von 186-188 °C zeigt.

Beispiel 11

5,49 g 3-Fluoranilin werden in 6,0 g konzentrierter Schwefelsäure und 120 ml Wasser gelöst und nach Zugabe von 10 g Eis bei 0° mit einer Lösung von 3,6 g Natriumnitrit in 10 ml Wasser diazotiert. Die Diazoniumsalzlösung wird dann mit Natriumacetat abgestumpft und bei 5 °C unter intensivem Rühren in eine Lösung von 17,2 g 4,5-Bis-(4-methoxyphenyl)-2-mercaptoimidazol in 700 ml Dimethylformamid gegeben. Man läßt 20 Minuten nachrühren, gießt in 2,5 l Wasser, extrahiert mit Essigester wäscht die organische Lösung mit Wasser, trocknet über Natriumsulfat und engt sie im Vakuum zur Trockne ein. Der Rückstand wird an 500 g neutralem Aluminiumoxid (Aktivitätsstufe II) chromatographiert. Mit Essigester/Hexan eluiert man 7,24 g 4,5-Bis-(4-methoxyphenyl)-2-(3-fluorphenylthio)-imidazol, das nach Umkristallisation aus Ether/Essigester einen Schmelzpunkt von 147-149 °C zeigt.

Beispiel 12

Zu einer Lösung von 8,11 g 4,5-Bis-(4-methoxyphenyl)-1-methoxymethyl-imidazol in einem Gemisch aus 50 ml absolutem Ether und 50 ml Benzol werden unter Argon und Rühren bei 0 °C 17,5 ml 1,5 N Butyllithium in Hexan, das mit 20 ml Benzol verdünnt wurde, getropft. Nach 20 Minuten tropft man die Lösung von 5,45 g Diphenyldisulfid in 25 ml absolutem Ether zu, wobei die Temperatur 20 °C nicht

überschreitet. Nach 2 Stunden Rühren bei Raumtemperatur extrahiert man die Lösung mit 2 N Natronlauge. Die organische Lösung wird im Vakuum zur Trockne eingeengt. Aus Hexan/Benzol kristallisieren 8,01 g 4,5-Bis-(4-methoxyphenyl)-1-methoxymethyl-2-phenylthio-imidazol vom Schmelzpunkt 88-90 °C.

Die Verbindung wird in 50 ml Eisessig gelöst, mit 10 ml 6 N Salzsäure versetzt und 6 Stunden am Rückfluß gekocht. Dann wird das Lösungsmittel im Vakuum abgezogen und der Rückstand zwischen Natriumbikarbonatlösung und Essigester verteilt. Man trocknet die organische Lösung über Natriumsulfat, engt sie im Vakuum zur Trockne ein, kristallisiert den Rückstand aus Hexan/Essigester und erhält so 6,27 g 4,5-Bis-(4-methoxyphenyl)-2-phenylthio-imidazol vom Schmelzpunkt 177-179 °C.

Herstellung des 4,5-Bis (4-methoxyphenyl)-1-methoxymethylimidazols :

Zu einer Lösung von 21 g 4,5-Bis-(4-methoxyphenyl)-imidazol in 75 ml absolutem Dimethylformamid werden anteilweise 3,6 g Natriumhydrid (50 % in Weißöl) gegeben. Man läßt 30 Minuten nachrühren und tropft dann 6,3 ml 1-Chlormethylmethylether in 35 ml Dimethylformamid zu. Nach 30 Minuten gießt man auf Eiswasser, nimmt das Produkt in Chloroform auf, wäscht die organische Lösung mit Wasser, trocknet sie über Natriumsulfat und engt die im Vakuum zur Trockne ein. Der Rückstand wird aus Benzol/Hexan kristallisiert. Man erhält so 18,31 g 4,5-Bis-(4-methoxyphenyl)-1-methoxymethyl-imidazol vom Schmelzpunkt 99-101 °C.

Beispiel 13

Zu einer Lösung von 3,65 g 4,5-Bis-(4-methoxyphenyl)-1-(2-tetrahydropyran-2-yl)-imidazol in einem Gemisch aus 50 ml absolutem Ether und 50 ml Benzol werden unter Rühren und Argon als Schutzgas bei − 5° bis 0 °C 7 ml ca. 1,5 N n-Butyl-lithium in Hexan, das mit 10 ml Benzol verdünnt wurde, getropft. Nach 20 Minuten tropft man die Lösung von 3,31 g 4,4'-Ditert.-butyl-diphenyl-disulfid in 25 ml absolutem Ether zu, wobei durch Kühlung die Temperatur zwischen −5° und 0 °C gehalten wird. Nach 6 Stunden Rühren bei Raumtemperatur gießt man in 800 ml Wasser, läßt das Produkt kristallisieren und saugt es ab. Man nimmt es in Ether auf, trocknet die Lösung über Natriumsulfat und engt bis zur beginnenden Kristallisation ein. Nach Umkristallisation aus Ether/Hexan erhält man 3,69 g 4,5-Bis-(4-methoxyphenyl)-2-(4-tert.-butyl-phenylthio)-1-(tetrahydropyran-2-yl)-imidazol vom Schmelzpunkt 162-164 °C.

Man nimmt die Verbindung in 25 ml Ethanol auf, versetzt mit 2 ml 6 N Salzsäure und erwärmt 10 Minuten auf dem Wasserbad. Dann zieht man das Lösungsmittel im Vakuum ab und kristallisert den Rückstand aus Ethanol/Ether. Man erhält so 3,08 g 4,5-Bis-(4-methoxyphenyl)-2-(4-tert.-butyl-phenylthio)-imidazol vom Schmelzpunkt 172-174 °C.

Herstellung des 4,5-Bis-(4-methoxyphenyl)-1-tetrahydropyran-2-yl)-imidazols :

In 250 ml absolutem 1,2-Dichlorethan werden 14,17 g 4,5-Bis-(4-methoxyphenyl)-imidazol suspendiert und mit 16 g 3,4-Dihydro-2H-pyran sowie 0,5 ml Zinntetrachlorid versetzt. Man erwärmt unter Rühren 12 Stunden zum Rückfluß, läßt erkalten und gießt in 500 ml kalte Natrumbikarbonatlösung. Die organische Phase wird abgetrennt, über Natriumsulfat getrocknet und im Vakuum eingeengt. Der Rückstand wird in Dichlormethan gelöst und einer Hexanfällung unterworfen. Der Niederschlag wird in Ether gelöst. Die Lösung wird mit Aktivkohle geklärt, im Vakuum eingeengt und zur Kristallisation gebracht. Man erhält so 15,03 g 4,5-Bis-(4-methoxyphenyl)-1-(tetrahydropyran-2-yl)-imidazol vom Schmelzpunkt 127-128 °C.

Beispiel 14

Zu einer Lösung von 3,65 g 4,5-Bis-(4-methoxyphenyl)-1-(2-tetrahydropyran-2-yl)-imidazol in einem Gemisch aus 50 ml absolutem Ether und 50 ml Benzol werden unter Rühren und Argon als Schutzgas bei −5° bis 0 °C 7 ml ca. 1,5 N n-Butyl-lithium in Hexan, das mit 10 ml Benzol verdünnt wurde, getropft. Nach 20 Minuten tropft man die Lösung von 3,56 g 3,3', 4,4'-Tetrachlor-diphenyl-disulfid in 30 ml absolutem Ether zu, wobei durch Kühlung die Temperatur zwischen −5 und 0 °C gehalten wird. Nach 6 Stunden Rühren bei Raumtemperatur gießt man in 800 ml Wasser, läßt das Produkt kristallisieren und saugt es ab. Man nimmt es in Ether auf, trocknet die Lösung über Natriumsulfat und engt bis zur beginnenden Kristallisation ein. Nach Umkristallisation aus Ether/Chloroform erhält man 4,68 g 4,5-Bis-(4-methoxyphenyl)-2-(3,4-dichlorphenylthio)-1-(tetrahydropyran-2-yl)-imidazol vom Schmelzpunkt 125-127 °C.

Man nimmt die Verbindung in 25 ml Ethanol auf, versetzt mit 5 ml etherische Salzsäure und erwärmt 10 Minuten auf dem Wasserbad. Dann zieht man das Lösungsmittel im Vakuum ab und verteilt den Rückstand zwischen Essigester und wässriger Natriumbikarbonatlösung, trocknet die organische Phase über Natriumsulfat, engt sie im Vakuum zur Trockne ein und kristallisert den Rückstand aus Ethanol/Ether. Man erhält so 3,41 g 4,5-Bis-(4-methoxyphenyl)-2-(3,4-dichlorphenylthio)-imidazol vom Schmelzpunkt 113-115 °C.

Beispiel 15

Zu einer Lösung von 3,65 g 4,5-Bis-(4-methoxyphenyl)-1-(2-tetrahydropyran-2-yl)-imidazol in einem Gemisch aus 50 ml absolutem Ether und 50 ml Benzol werden unter Rühren und Argon als Schutzgas bei – 5° bis 0 °C 7 ml ca. 1,5 N n-Butyl-lithium in Hexan, das mit 10 ml Benzol verdünnt wurde, getropft. Nach 20 Minuten tropft man die Lösung von 3,76 g 4,4'-diphenyl-disulfid in 300 ml absolutem Ether zu, wobei durch Kühlung die Temperatur zwischen – 5° und 0 °C gehalten wird. Nach 6 Stunden Rühren bei Raumtemperatur gießt man in 800 ml Wasser, läßt das Produkt kristallisieren und saugt es ab. Man nimmt es in Ether auf, trocknet die Lösung über Natriumsulfat und engt bis zur beginnenden Kristallisation ein. Nach Umkristallisation aus Ether/Chloroform erhält man 4,27 g 4,5-Bis-(4-methoxyphenyl)-2-(4-bromphenylthio)-1-(tetrahydropyran-2-yl)-imidazol vom Schmelzpunkt 192-194 °C.

Man nimmt die Verbindung in 25 ml Ethanol auf, versetzt mit 5 ml etherische Salzsäure und erwärmt 10 Minuten auf dem Wasserbad. Dann zieht man das Lösungsmittel im Vakuum ab und verteilt den Rückstand zwischen Essigester und wässriger Natriumbikarbonatlösung, trocknet die organische Phase über Natriumsulfat, engt sie im Vakuum zur Trockne ein und kristallisert den Rückstand aus Ethanol/Ether. Man erhält so 3,19 g 4,5-Bis-(4-methoxyphenyl)-2-(4-bromphenylthio)-imidazol vom Schmelzpunkt 138-140 °C.

Beispiel 16

Zu einer Lösung von 3,65 g 4,5-Bis-(4-methoxyphenyl)-1-(2-tetrahydropyran-2-yl)-imidazol in einem Gemisch aus 50 ml absolutem Ether und 50 ml Benzol werden unter Rühren und Argon als Schutzgas bei – 5° bis 0 °C 7 ml ca. 1,5 N n-Butyl-lithium in Hexan, das mit 10 ml Benzol verdünnt wurde, getropft. Nach 20 Minuten tropft man die Lösung von 2,87 g 4,4'-Dichlor-diphenyl-disulfid in 50 ml absolutem Ether zu, wobei durch Kühlung die Temperatur zwischen – 5° und 0 °C gehalten wird. Nach 6 Stunden Rühren bei Raumtemperatur gießt man in 800 ml Wasser, läßt das Produkt kristallisieren und saugt es ab. Man nimmt es in Ether auf, trocknet die Lösung über Natriumsulfat und engt bis zur beginnenden Kristallisation ein. Nach Umkristallisation aus Ether/Chloroform erhält man 4,68 g 4,5-Bis-(4-methoxyphenyl)-2-(4-chlorphenylthio)-1-(tetrahydropyran-2-yl)-imidazol vom Schmelzpunkt 202-204 °C.

Man nimmt die Verbindung in 25 ml Ethanol auf, versetzt mit 5 ml etherische Salzsäure und erwärmt 10 Minuten auf dem Wasserbad. Dann zieht man das Lösungsmittel im Vakuum ab und verteilt den Rückstand zwischen Essigester und wässriger Natriumbikarbonatlösung, trocknet die organische Phase über Natriumsulfat, engt sie im Vakuum zur Trockne ein und kristallisert den Rückstand aus Ethanol/Ether. Man erhält so 3,41 g 4,5-Bis-(4-methoxyphenyl)-2-(4-chlorphenylthio)-imidazol vom Schmelzpunkt 148-150 °C.

Beispiel 17

Zu einer Lösung von 3,65 g 4,5-Bis-(4-methoxyphenyl)-1-(2-tetrahydropyran-2-yl)-imidazol in einem Gemisch aus 50 ml absolutem Ether und 50 ml Benzol werden unter Rühren und Argon als Schutzgas bei – 5° bis 0 °C 7 ml ca. 1,5 N n-Butyllithium in Hexan, das mit 10 ml Benzol verdünnt wurde, getropft. Nach 20 Minuten tropft man die Lösung von 2,71 g 4,4'-Dimethyl-diphenyl-disulfid in 50 ml absolutem Ether zu, wobei durch Kühlung die Temperatur zwischen – 5° und 0 °C gehalten wird. Nach 6 Stunden Rühren bei Raumtemperatur gießt man in 800 ml Wasser, nimmt das Produkt in Essigester auf, trocknet die organische Lösung über Natriumsulfat und engt im Vakuum zur Trockne ein. Der Rückstand wird durch Chromatographie an Kieselgel mit Hexan/Aceton 3 : 2 gereinigt. Nach Umkristallisation aus Ether erhält man 3,97 g 4,5-Bis-(4-methoxyphenyl)-2-(4-methylphenylthio)-1-(tetrahydropyran-2-yl)-imidazol vom Schmelzpunkt 192 °C.

Man nimmt die Verbindung in 25 ml Ethanol auf, versetzt mit 5 ml etherische Salzsäure und erwärmt 10 Minuten auf dem Wasserbad. Dann zieht man das Lösungsmittel im Vakuum ab verteilt den Rückstand zwischen Essigester und wässriger Natriumbikarbonatlösung, trocknet die organische Phase über Natriumsulfat, engt sie im Vakuum zur Trockne ein und kristallisiert den Rückstand aus Ethanol/Ether. Man erhält so das 4,5-Bis-(4-methoxyphenyl)-2-(4-methylphenylthio)-imidazol vom Schmelzpunkt 181 °C.

Beispiel 18

Zu einer Lösung von 3,65 g 4,5-Bis-(4-methoxyphenyl)-1-(2-tetrahydropyran-2-yl)-imidazol in einem Gemisch aus 50 ml absolutem Ether und 50 ml Benzol werden unter Rühren und Argon als Schutzgas bei – 5° bis 0 °C 7 ml ca. 1,5 N n-Butyllithium in Hexan, das mit 10 ml Benzol verdünnt wurde, getropft. Nach 20 Minuten tropft man die Lösung von 2,87 g 4,4'-Dimethoxy-diphenyl-disulfid in 40 ml absolutem Ether zu, wobei durch Kühlung die Temperatur zwischen – 5° und 0 °C gehalten wird. Nach 6 Stunden Rühren bei Raumtemperatur gießt man in 800 ml Wasser, nimmt das Produkt in Essigester auf, trocknet die organische Lösung über Natriumsulfat und engt im Vakuum zur Trockne ein. Der Rückstand wird durch Chromatographie an Kieselgel mit Hexan/Aceton 3 : 2 gereinigt. Nach Umkristallisation aus Ether erhält man 4,27 g 4,5-Bis-(4-methoxyphenyl)-2-(4-methoxyphenylthio)-1-(tetrahydropyran-2-yl)-imidazol vom Schmelzpunkt 152 °C.

Man nimmt die Verbindung in 25 ml Ethanol auf, versetzt mit 5 ml etherische Salzsäure und erwärmt 10 Minuten auf dem Wasserbad. Dann zieht man das Lösungsmittel im Vakuum ab verteilt den Rückstand zwischen Essigester und wässriger Natriumsulfat, engt sie im Vakuum zur Trockne ein und kristallisiert den Rückstand aus Ether. Man erhält so 3,10 g 4,5-Bis-(4-methoxyphenyl)-2-(4-methoxyphenylthio)-imidazol vom Schmelzpunkt 148 °C.

Beispiel 19

Zu einer Lösung von 3,65 g 4,5-Bis-(4-methoxyphenyl)-1-(2-tetrahydropyran-2-yl)-imidazol in einem Gemisch aus 50 ml absolutem Ether und 50 ml Benzol werden unter Rühren und Argon als Schutzgas bei – 5° bis 0 °C 7 ml ca. 1,5 N n-Butyl-lithium in Hexan, das mit 10 ml Benzol verdünnt wurde, getropft. Nach 20 Minuten tropft man die Lösung von 2,87 g 2,2'-Dimethoxy-diphenyl-disulfid in 50 ml absolutem Ether zu, wobei durch Kühlung die Temperatur zwischen – 5° und 0 °C gehalten wird. Nach 6 Stunden Rühren bei Raumtemperatur gießt man in 800 ml Wasser, nimmt das Produkt in Essigester auf, trocknet die organische Lösung über Natriumsulfat und engt im Vakuum zur Trockne ein. Der Rückstand wird durch Chromatographie an Kieselgel mit Hexan/Aceton 3 : 2 gereinigt. Nach Umkristallsiation aus Ether erhält man 4,13 g 4,5-Bis-(4-methoxyphenyl)-2-(2-methoxyphenylthio)-1-(tetrahydropyran-2-yl)-imidazol vom Schmelzpunkt 166 °C.

Man nimmt die Verbindung in 25 ml Ethanol auf, versetzt mit 5 ml etherische Salzsäure und erwärmt 10 Minuten auf dem Wasserbad. Dann zieht man das Lösungsmittel im Vakuum ab und kristallisiert den Rückstand aus Toluol/Petrolether. Man erhält so 2,93 g 4,5-Bis-(4-methoxyphenyl)-2-(2-methoxyphenylthio)-imidazol vom Schmelzpunkt 161 °C.

Beispiel 20

Zu einer Lösung von 3,65 g 4,5-Bis-(4-methoxyphenyl)-1-(2-tetrahydropyran-2-yl)-imidazol in einem Gemisch aus 50 ml absolutem Ether und 50 ml Benzol werden unter Rühren und Argon als Schutzgas bei – 5° bis 0 °C 7 ml ca. 1,5 N n-Butyl-lithium in Hexan, das mit 10 ml Benzol verdünnt wurde, getropft. Nach 20 Minuten tropft man die Lösung von 2,87 g 3,3'-Dimethoxy-diphenyl-disulfid in 30 ml absolutem Ether zu, wobei durch kühlung die Temperatur zwischen – 5° und 0 °C gehalten wird. Nach 6 Stunden Rühren bei Raumtemperatur gießt man in 800 ml Wasser, nimmt das Produkt in Essigester auf, trocknet die organische Lösung über Natriumsulfat und engt im Vakuum zur Trockne ein. Der Rückstand wird durch Chromatographie an Kieselgel mit Hexan/Aceton 3 : 2 gereinigt. Nach Umkristallisation aus Ether erhält man 3,90 g 4,5-Bis-(4-methoxyphenyl)-2-(3-methoxyphenylthio)-1-(tetrahydropyran-2-yl)-imidazol vom Schmelzpunkt 123 °C.

Man nimmt die Verbindung in 25 ml Ethanol auf, versetzt mit 5 ml etherische Salzsäure und erwärmt 10 Minuten auf dem Wasserbad. Dann zieht man das Lösungsmittel im Vakuum ab und verteilt den Rückstand zwischen Essigester und wässriger Natriumbikarbonatlösung, trocknet die organische Phase über Natriumsulfat, engt sie im Vakuum zur Trockne ein und kristallisert den Rückstand aus Ethanol/Ether. Man erhält so 3,08 g 4,5-Bis-(4-methoxyphenyl)-2-(3-methoxyphenylthio)-imidazol vom Schmelzpunkt 101 °C.

Beispiel 21

Zu einer Suspension aus 14,7 g 4,5-Bis-(4-methoxyphenyl)-1-methylimidazol in 150 ml eines Gemisches aus absolutem Äther/Benzol 2 : 1 werden unter Kühlung 34,5 ml einer ca. 1,6 N Lösung von Lithiumbutyl in Hexan unter Rühren und Argonabdeckung getropft, wobei Feststoff in Lösung geht. Man läßt 30 Minuten nachrühren und tropft dann 10,9 g Diphenyldisulfid in 50 ml absolutem Ether gelöst zu, wobei man die Temperatur durch Kühlung nicht über 40° ansteigen läßt. Nach 30 Minuten Nachrühren wäscht man die Lösung mit 2 N Natronlauge und Wasser, trocknet sie über Natriumsulfat und engt sie im Vakuum zur Trockne ein. Der Rückstand wird aus Ether kristallisiert, und man erhält so 17,4 g 4,5-Bis-(4-methoxyphenyl)-2-phenylthio-1-methylimidazol vom Schmelzpunkt 115-117 °C.

Herstellung des 4,5-Bis-(4-methoxyphenyl)-1-methylimidazols :

Zu einer Lösung von 10,5 g 4,6-Bis-(4-methoxyphenyl)-imidazol in 50 ml absolutem Dimethylformamid werden anteilweise 1,8 g Natriumhydrid (50 % in Weißöl) gegeben. Man läßt 30 Minuten nachrühren und tropft dann 5,35 g Methylkiodid in 50 ml Dimethylformamid zu. Nach 60 Minuten gießt man in Eiswasser, nimmt das Produkt in Essigester auf, trocknet die organische Lösung über Natriumsulfat und engt sie im Vakuum ein. Der Rückstand wird aus Benzol umkristallisiert. Man erhält so 9,59 g 4,5-Bis-(4-methoxyphenyl)-1-methylimidazol vom Schmelzpunkt 122-123 °C.

Beispiel 22

Zu einer Lösung von 3,89 g 4,5-Bis-(4-methoxyphenyl)-2-phenylthio-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein. Der Rückstand wird aus Ether/Hexan kristallisiert. Nach Umkristallisation aus Ether/Hexan erhält man 3,36 g 4,5-Bis-(4-methoxyphenyl)-2-phenylsulfinyl-imidazol vom Schmelzpunkt 188-190 °C.

Beispiel 23

Zu einer Lösung von 3,89 g 4,5-Bis-(4-methoxyphenyl)-2-phenylthio-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein. Der Rückstand wird aus Ether/Methylenchlorid kristallisiert und man erhält 3,27 g 4,5-Bis-(4-methoxyphenyl)-2-phenylsulfonyl-imidazol vom Schmelzpunkt 106 °C.

Beispiel 24

Zu einer Lösung von 3,95 g 4,5-Bis-(4-methoxyphenyl)-2-(2-thienylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,47 g 4,5-Bis-(4-methoxyphenyl)-2-(2-thienylsulfinyl-imidazol als amorphen Schaum.

$C_{21}H_{18}N_2O_3S_2$ (410,52)

| | | | | |
|---|---|---|---|---|
| Ber.: | 61,44 % C | 4,20 % H | 6,82 % N | 15,62 % S |
| Gef.: | 61,27 % C | 4,29 % H | 6,71 % N | 15,50 % S |

Beispiel 25

Zu einer Lösung von 3,95 g 4,5-Bis-(4-methoxyphenyl)-2-(2-thienylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein. Der Rückstand wird aus Ether kristallisiert und man erhält 3,19 g 4,5-Bis-(4-methoxyphenyl)-2-(2-thienylsulfonyl)-imidazol vom Schmelzpunkt 179 °C.

Beispiel 26

Zu einer Lösung von 3,95 g 4,5-Bis-(4-methoxyphenyl)-2-(2-thiazolylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird and 150 g Kieselgel mit Aceton/Hexan 2 : 3 chromatographiert und man erhält 3,28 g 4,5-Bis-(4-methoxyphenyl)-2-(2-thiazolylsulfinyl)-imidazol als amorphen Schaum.

$C_{20}H_{17}N_3O_3S_2$ (411,50)

| | | | | |
|---|---|---|---|---|
| Ber.: | 58,38 % C | 4,16 % H | 10,21 % N | 15,58 % S |
| Gef.: | 58,24 % C | 4,37 % H | 10,13 % N | 15,49 % S |

Beispiel 27

Zu einer Lösung von 3,96 g 4,5-Bis-(4-methoxyphenyl)-2-(2-thiazolylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,39 g 4,5-Bis-(4-methoxyphenyl)-2-(2-thiazolylsulfonyl)-imidazol vom Schmelzpunkt 219 °C.

Beispiel 28

Zu einer Lösung von 4,57 g 4,5-Bis-(4-methoxyphenyl)-2-(3,4-dichlorphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan 2 : 3 chromatographiert und man erhält 4,19 g 4,5-Bis-(4-methoxyphenyl)-2-(3,4-dichlorphenylsulfinyl)-imidazol als amorphen Schaum.

$C_{23}H_{18}Cl_2N_2O_3S$ (473,38)

| | | | | | |
|---|---|---|---|---|---|
| Ber. : | 58,36 % C | 3,83 % H | 14,98 % Cl | 5,92 % N | 6,77 % S |
| Gef. : | 58,30 % C | 3,91 % H | 14,88 % Cl | 5,84 % N | 6,66 % S |

Beispiel 29

Zu einer Lösung von 4,26 g 4,5-Bis-(4-methoxyphenyl)-2-(3,4-dichlorphenylthio)-imidazol in 100 ml Dichlormethan tropft man die Lösung von 2,164 g 3-Chlorperbenzoesäure (80 %) in 140 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,19 g 4,5-Bis-(4-methoxyphenyl)-2-(3,4-dichlorphenylsulfonyl)-imidazol als amorphen Schaum.

$C_{23}H_{18}Cl_2N_2O_4S$ (489,38)

| | | | | | |
|---|---|---|---|---|---|
| Ber. : | 56,45 % C | 3,71 % H | 14,49 % Cl | 5,72 % N | 6,55 % S |
| Gef. : | 56,29 % C | 3,82 % H | 14,40 % Cl | 5,67 % N | 6,48 % S |

Beispiel 30

Zu einer Lösung von 4,07 g 4,5-Bis-(4-methoxyphenyl)-2-(4-fluorphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,86 g 4,5-Bis-(4-methoxyphenyl)-2-(4-fluorphenylsulfinyl)-imidazol als amorphen Schaum.

$C_{23}H_{19}FN_2O_3S$ (422,49)

| | | | | | |
|---|---|---|---|---|---|
| Ber. : | 65,39 % C | 4,53 % H | 4,50 % F | 6,63 % N | 7,59 % S |
| Gef. : | 65,27 % C | 4,65 % H | 4,44 % F | 6,54 % N | 7,48 % S |

Beispiel 31

Zu einer Lösung von 4,07 g 4,5-Bis-(4-methoxyphenyl)-2-(4-fluorphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,98 g 4,5-Bis-(4-methoxyphenyl)-2-(4-fluorphenylsulfonyl)-imidazol als amorphen Schaum.

$C_{23}H_{19}FN_2O_4S$ (438,49)

| | | | | | |
|---|---|---|---|---|---|
| Ber. : | 63,00 % C | 4,37 % H | 4,33 % F | 6,39 % N | 7,31 % S |
| Gef. : | 63,13 % C | 4,46 % H | 4,29 % F | 6,33 % N | 7,24 % S |

Beispiel 32

Zu einer Lösung von 4,07 g 4,5-Bis-(4-methoxyphenyl)-2-(3-fluorphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,04 g 4,5-Bis-(4-methoxyphenyl)-2-(3-fluorphenylsulfinyl)-imidazol als amorphen Schaum.

$C_{23}H_{19}FN_2O_3S$ (422,49)

| | | | | | |
|---|---|---|---|---|---|
| Ber. : | 65,39 % C | 4,53 % H | 4,50 % F | 6,63 % N | 7,59 % S |
| Gef. : | 65,31 % C | 4,62 % H | 4,50 % F | 6,54 % N | 7,48 % S |

Beispiel 33

Zu einer Lösung von 4,07 g 4,5-Bis-(4-methoxyphenyl)-2-(3-fluorphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,99 g 4,5-Bis-(4-methoxyphenyl)-2-(3-fluorphenylsulfonyl)-imidazol als amorphen Schaum.

| | | | | | |
|---|---|---|---|---|---|
| $C_{23}H_{19}FN_2O_4S$ | (438,49) | | | | |
| Ber.: | 63,00 % C | 4,37 % H | 4,33 % F | 6,39 % N | 7,31 % S |
| Gef.: | 62,88 % C | 4,45 % H | 4,26 % F | 6,14 % N | 7,19 % S |

## Beispiel 34

Zu einer Lösung von 4,07 g 4,5-Bis-(4-methoxyphenyl)-2-(2-fluorphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,49 g 4,5-Bis-(4-methoxyphenyl)-2-(2-fluorphenylsulfinyl)-imidazol als amorphen Schaum.

| | | | | | |
|---|---|---|---|---|---|
| $C_{23}H_{19}FN_2O_3S$ | (422,49) | | | | |
| Ber.: | 65,39 % C | 4,53 % H | 4,50 % F | 6,63 % N | 7,59 % S |
| Gef.: | 65,28 % C | 4,61 % H | 4,55 % F | 6,56 % N | 7,43 % S |

## Beispiel 35

Zu einer Lösung von 4,07 g 4,5-Bis-(4-methoxyphenyl)-2-(2-fluorphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,94 g 4,5-Bis-(4-methoxyphenyl)-2-(2-fluorphenylsulfonyl)-imidazol als amorphen Schaum.

| | | | | | |
|---|---|---|---|---|---|
| $C_{23}H_{19}FN_2O_4S$ | (438,49) | | | | |
| Ber.: | 63,00 % C | 4,37 % H | 4,33 % F | 6,39 % N | 7,31 % S |
| Gef.: | 62,90 % C | 4,45 % H | 4,27 % F | 6,43 % N | 7,32 % S |

## Beispiel 36

Zu einer Lösung von 4,19 g 4,5-Bis-(4-methoxyphenyl)-2-(2-methoxyphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,86 g 4,5-Bis-(4-methoxyphenyl)-2-(2-methoxyphenylsulfinyl)-imidazol als amorphen Schaum.

| | | | | |
|---|---|---|---|---|
| $C_{24}H_{22}N_2O_4S$ | (434,53) | | | |
| Ber.: | 66,34 % C | 5,10 % H | 6,45 % N | 7,38 % S |
| Gef.: | 66,23 % C | 5,16 % H | 6,39 % N | 7,30 % S |

## Beispiel 37

Zu einer Lösung von 4,19 g 4,5-Bis-(4-methoxyphenyl)-2-(2-methoxyphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,72 g 4,5-Bis-(4-methoxyphenyl)-2-(2-methoxyphenylsulfonyl)-imidazol als amorphen Schaum.

| | | | | |
|---|---|---|---|---|
| $C_{24}H_{22}N_2O_5S$ | (450,53) | | | |
| Ber.: | 63,99 % C | 4,92 % H | 6,22 % N | 7,12 % S |
| Gef.: | 64,07 % C | 5,04 % H | 6,17 % N | 7,02 % S |

## Beispiel 38

Zu einer Lösung von 4,19 g 4,5-Bis-(4-methoxyphenyl)-2-(4-methoxyphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,92 g 4,5-Bis-(4-methoxyphenyl)-2-(4-methoxyphenylsulfinyl)-imidazol als amorphen Schaum.

$C_{24}H_{22}N_2O_4S$ (434,53)
Ber.: 66,34 % C 5,10 % H 6,45 % N 7,38 % S
Gef.: 66,27 % C 5,17 % H 6,40 % N 7,42 % S

Beispiel 39

Zu einer Lösung von 4,19 g 4,5-Bis-(4-methoxyphenyl)-2-(4-methoxyphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,05 g 4,5-Bis-(4-methoxyphenyl)-2-(4-methoxyphenylsulfonyl)-imidazol als amorphen Schaum.

$C_{24}H_{22}N_2O_5S$ (450,53)
Ber.: 63,99 % C 4,92 % H 6,22 % N 7,12 % S
Gef.: 63,86 % C 4,99 % H 6,25 % N 7,06 % S

Beispiel 40

Zu einer Lösung von 4,19 g 4,5-Bis-(4-methoxyphenyl)-2-(3-methoxyphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,96 g 4,5-Bis-(4-methoxyphenyl)-2-(3-methoxyphenylsulfinyl)-imidazol als amorphen Schaum.

$C_{24}H_{22}N_2O_4S$ (434,53)
Ber.: 66,34 % C 5,10 % H 6,45 % N 7,38 % S
Gef.: 66,24 % C 5,15 % H 6,38 % N 7,33 % S

Beispiel 41

Zu einer Lösung von 4,19 g 4,5-Bis-(4-methoxyphenyl)-2-(3-methoxyphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,02 g 4,5-Bis-(4-methoxyphenyl)-2-(3-methoxyphenylsulfonyl)-imidazol als amorphen Schaum.

$C_{24}H_{22}N_2O_5S$ (450,53)
Ber.: 63,99 % C 4,92 % H 6,22 % N 7,12 % S
Gef.: 63,87 % C 5,03 % H 6,17 % N 7,09 % S

Beispiel 42

Zu einer Lösung von 4,25 g 4,5-Bis-(4-methoxyphenyl)-2-(2,4-difluorphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,26 g 4,5-Bis-(4-methoxyphenyl)-2-(2,4-difluorphenylsulfinyl)-imidazol als amorphen Schaum.

$C_{23}H_{19}F_2N_2O_3S$ (440,47)
Ber.: 62,72 % C 4,12 % H 8,63 % F 6,36 % N 7,28 % S
Gef.: 62,59 % C 4,23 % H 8,55 % F 6,28 % N 7,21 % S

Beispiel 43

Zu einer Lösung von 4,25 g 4,5-Bis-(4-methoxyphenyl)-2-(2,4-difluorphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie in Vakuum zur Trockne ein.

Der Rückstand wird an 150 g Keisegel mit Aceton/Hexan chromatographiert und man erhält 3,41 g 4,5-Bis-(4-methoxyphenyl)-2-(2,4-difluorphenylsulfonyl)-imidazol als amorphen Schaum.

$C_{23}H_{18}F_2N_2O_4S$ (456,47)

| | | | | | |
|---|---|---|---|---|---|
| Ber.: | 60,52 % C | 3,97 % H | 8,32 % F | 6,14 % B | 7,02 % S |
| Gef.: | 60,61 % C | 4,07 % H | 8,22 % F | 6,03 % N | 6,90 % S |

Beispiel 44

Zu einer Lösung von 4,57 g 4,5-Bis-(4-methoxyphenyl)-2-(4-trifluormethylphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wächst die Lösung mit Natriumbikarbonatlösung, trocknet sie über Natriumsulfat und engt sie in Vakuum zur Trockne ein.

Der Rückstand wird an 150 g Kieselgel mit Aceton/Hexan chromatographiert und man erhält 4,26 g 4,5-Bis-(4-methoxyphenyl)-2-(4-trifluormethylphenylsulfinyl)-imidazol als amorphen Schaum.

$C_{24}H_{19}F_3N_2O_3S$ (472,49)

| | | | | | |
|---|---|---|---|---|---|
| Ber.: | 61,01 % C | 4,05 % H | 12,6 % F | 5,93 % N | 6,79 % S |
| Gef.: | 60,90 % C | 4,13 % H | 11,97 % F | 6,02 % N | 6,71 % S |

Beispiel 45

Zu einer Lösung von 4,57 g 4,5-Bis-(4-methoxyphenyl)-2-(4-trifluormethylpenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wächst die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,33 g 4,5-Bis-(4-methoxyphenyl)-2-(4-trifluormethylphenylsulfonyl)-imidazol als amorphen Schaum.

$C_{24}H_{19}F_3N_2O_4S$ (488,49)

| | | | | | |
|---|---|---|---|---|---|
| Ber.: | 59,01 % C | 3,92 % H | 11,67 % F | 5,73 % N | 6,56 % S |
| Gef.: | 58,89 % C | 4,00 % H | 11,61 % F | 5,68 % N | 6,59 % S |

Beispiel 46

Zu einer Lösung von 4,67 g 4,5-Bis-(4-methoxyphenyl)-2-(4-bromphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,36 g 4,5-Bis-(4-methoxyphenyl)-2-(4-bromphenylsulfinyl)-imidazol als amorphen Schaum.

$C_{23}H_{19}BrN_2O_3S$ (483,39)

| | | | | | |
|---|---|---|---|---|---|
| Ber.: | 57,15 % C | 3,96 % H | 16,53 % Br | 5,80 % N | 6,63 % S |
| Gef.: | 57,07 % C | 4,07 % H | 16,41 % Br | 5,72 % N | 6,58 % S |

Beispiel 47

Zu einer Lösung von 4,67 g 4,5-Bis-(4-methoxyphenyl)-2-(4-bromphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,41 g 4,5-Bis-(4-methoxyphenyl)-2-(4-bromphenylsulfonyl)-imidazol als amorphen Schaum.

$C_{23}H_{19}BrN_2O_4S$ (499,39)

| | | | | | |
|---|---|---|---|---|---|
| Ber.: | 55,32 % C | 3,83 % H | 16,00 % Br | 5,61 % N | 6,42 % S |
| Gef.: | 55,27 % C | 3,90 % H | 15,79 % Br | 5,54 % N | 6,36 % S |

Beispiel 48

Zu einer Lösung von 4,23 g 4,5-Bis-(4-methoxyphenyl)-2-(4-chlorphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,90 g 4,5-Bis-(4-methoxyphenyl)-2-(4-chlorphenylsulfinyl)-imidazol als amorphen Schaum.

$C_{23}H_{19}ClN_2O_3S$ (438,94)
Ber.: 62,94 % C 4,36 % H 8,08 % Cl 6,38 % N 7,30 % S
Gef.: 62,87 % C 4,42 % H 8,00 % Cl 6,26 % N 7,23 % S

Beispiel 49

Zu einer Lösung von 4,23 g 4,5-Bis-(4-methoxyphenyl)-2-(4-chlorphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,93 g 4,5-Bis-(4-methoxyphenyl)-2-(4-chlorphenylsulfonyl)-imidazol als amorphen Schaum.

$C_{23}H_{19}ClN_2O_4S$ (454,93)
Ber.: 60,72 % C 4,21 % H 7,79 % Cl 6,16 % N 7,05 % S
Gef.: 60,61 % C 4,28 % H 7,68 % Cl 6,09 % N 6,96 % S

Beispiel 50

Zu einer Lösung von 4,03 g 4,5-Bis-(4-methoxyphenyl)-2-(4-methylphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatograhiert und man erhält 3,71 g 4,5-Bis-(4-methoxyphenyl)-2-(4-methylphenylsulfinyl)-imidazol als amorphen Schaum.

$C_{24}H_{22}N_2O_3S$ (418,52)
Ber.: 68,88 % C 5,30 % H 6,69 % N 7,66 % S
Gef.: 68,75 % C 5,37 % H 6,60 % N 7,61 % S

Beispiel 51

Zu einer Lösung von 4,03 g 4,5-Bis-(4-methoxyphenyl)-2-(4-methylphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,96 g 4,5-Bis-(4-methoxyphenyl)-2-(4-methylphenylsulfonyl)-imidazol als amorphen Schaum.

$C_{24}H_{22}N_2O_4S$ (434,52)
Ber.: 66,34 % C 5,10 % H 6,45 % N 7,38 % S
Gef.: 66,29 % C 5,18 % H 6,45 % N 7,28 % S

Beispiel 52

Zu einer Lösung von 4,45 g 4,5-Bis-(4-methoxyphenyl)-2-(4-tert.-butylphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,17 g 4,5-Bis-(4-methoxyphenyl)-2-(4-tert.-butylphenylsulfinyl)-imidazol als amorphen Schaum.

$C_{27}H_{28}N_2O_3S$ (460,60)
Ber.: 70,41 % C 6,13 % H 6,08 % N 6,96 % S
Gef.: 70,36 % C 6,17 % H 6,01 % N 6,66 % S

Beispiel 53

Zu einer Lösung von 4,45 g 4,5-Bis-(4-methoxyphenyl)-2-(4-tert.-butylphenylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,28 g 4,5-Bis-(4-methoxyphenyl)-2-(4-tert.-butylphenylsulfonyl)-imidazol als amorphen Schaum.

$C_{27}H_{22}N_2O_4S$ (476,60)
Ber. : 68,04 % C 5,92 % H 5,88 % N 6,73 % S
Gef. : 67,92 % C 6,03 % H 5,69 % N 6,66 % S

Beispiel 54

Zu einer Lösung von 4,03 g 4,5-Bis-(4-methoxyphenyl)-2-phenylthio-1-methyl-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 200 g Kieselgel mit Essigester/Cyclohexan 2 : 3 chromatograhiert und man erhält 3,05 g 4,5-Bis-(4-methoxyphenyl)-2-phenylsulfinyl-1-methyl-imidazol, das nach Umkristallisation aus Dichlormethan/Ether einen Schmelzpunkt von 114-115 °C hat.

Beispiel 55

Zu einer Lösung von 4,03 g 4,5-Bis-(4-methoxyphenyl)-2-phenylthio-1-methyl-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält nach Umkristallisation aus Dichlormethan/Cyclohexan 3,19 g 4,5-Bis-(4-methoxyphenyl)-2-(2-thiazolylsulfonyl)-imidazol vom Schmelzpunkt 148-150 °C.

Beispiel 56

Zu einer Lösung von 3,90 g 4,5-Bis-(4-methoxyphenyl)-2-(2-pyridylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,42 g 4,5-Bis-(4-methoxyphenyl)-2-(2-pyridylsulfinyl)-imidazol als amorphen Schaum.

$C_{22}H_{19}N_3O_3S$ (405,48)
Ber. : 65,17 % C 4,72 % H 10,36 % N 7,91 % S
Gef. : 65,07 % C 4,83 % H 10,21 % N 7,79 % S

Beispiel 57

Zu einer Lösung von 3,90 g 4,5-Bis-(4-methoxyphenyl)-2-(2-pyridylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,44 g 4,5-Bis-(4-methoxyphenyl)-2-(2-pyridylsulfonyl)-imidazol als amorphen Schaum.

$C_{22}H_{19}N_3O_4S$ (421,48)
Ber. : 62,69 % C 4,54 % H 9,97 % N 7,61 % S
Gef. : 62,66 % C 4,66 % H 9,89 % N 7,54 % S

Beispiel 58

Zu einer Lösung von 3,91 g 4,5-Bis-(4-methoxyphenyl)-2-(2-pyrimidinylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,52 g 4,5-Bis-(4-methoxyphenyl)-2-(2-pyrimidinylsulfinyl)-imidazol als amorphen Schaum.

$C_{21}H_{18}N_4O_3S$ (406,47)
Ber. : 62,06 % C 4,46 % H 13,78 % N 7,89 % S
Gef. : 62,15 % C 4,40 % H 13,70 % N 7,79 % S

Beispiel 59

Zu einer Lösung von 3,91 g 4,5-Bis-(4-methoxyphenyl)-2-(2-pyrimidinylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumslfat und engt sie im Vakuum zur Trockne ein.

Der Rückstand wird an 150 g Kieselgel mit Aceton/Hexan chromatographiert und man erhält 3,54 g 4,5-Bis-(4-methoxyphenyl)-2-(2-pyrimidinylsulfonyl)-imidazol als amorphen Schaum.

$C_{21}H_{18}N_4O_4S$ (422,47)

| | | | | |
|---|---|---|---|---|
| Ber. : | 59,71 % C | 4,29 % H | 13,26 % N | 7,59 % S |
| Gef. : | 59,62 % C | 4,40 % H | 13,15 % N | 7,49 % S |

Beispiel 60

In 150 ml absolutem Dimethylformamid werden 6,43 g 4,5-Bis-(4-chlorphenyl)-2-mercaptoimidazol gelöst und mit 0,6 g Natriumhydrid (80 %ig in Weißöl) versetzt. Man läßt 30 Minuten nachrühren, tropft dann 4,25 g 2-Jodthiophen in 30 ml Dimethylformamid zu und erhitzt 16 Stunden unter Argon am Rückfluß. Dann gießt man die Lösung in 600 ml Eiswaser, extrahiert das Produkt mit Essigester, trocknet die organische Lösung über Natriumsulfat und engt sie im Vakuum zur Trockne ein. Der Rückstand wird aus Ether umkristallisiert und man erhält 7,02 g 4,5-Bis-(4-chlorphenyl)-2-(2-thienylthio)-imidazol vom Schmelzpunkt 219 °C.

Beispiel 61

Zu einer Lösung von 4,90 g 4,5-Bis-(4-chlorphenyl)-2-(2-thienylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,83 g 4,5-Bis-(4-chlorphenyl)-2-(2-thienylsulfinyl)-imidazol als amorphen Schaum.

$C_{19}H_{22}Cl_2N_2OS$ (455,47)

| | | | | | |
|---|---|---|---|---|---|
| Ber. : | 50,11 % C | 2,66 % H | 15,57 % Cl | 6,15 % N | 14,08 % S |
| Gef. : | 49,98 % C | 2,77 % H | 15,44 % Cl | 6,08 % N | 13,97 % S |

Beispiel 62

Zu einer Lösung von 4,40 g 4,5-Bis-(4-chlorphenyl)-2-(2-thienylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,83 g 4,5-Bis-(4-chlorphenyl)-2-(2-thienylsulfonyl)-imidazol als amorphen Schaum.

$C_{19}H_{12}Cl_2N_2O_2S_2$ (471,47)

| | | | | | |
|---|---|---|---|---|---|
| Ber. : | 48,40 % C | 2,57 % H | 15,04 % Cl | 5,94 % N | 13,60 % S |
| Gef. : | 48,21 % C | 2,68 % H | 15,12 % Cl | 5,82 % N | 13,51 % S |

Beispiel 63

In 100 ml absolutem Dimethylformamid werden 3,12 g 4,5-Bis-(4-methoxyphenyl)-2-mercaptoimidazol gelöst und mit 0,6 g Natriumhydrid (80 %ig in Weißöl) versetzt. Man läßt 30 Minuten nachrühren, gibt dann 1,52 g 4-Chlorpyridin-Hydrochlorid und läßt 16 Stunden unter Argon und Rückfluß kochen. Dann gießt man die Lösung in 300 ml Eiswasser, extrahiert das Produkt mit Essigester, trocknet die organische Lösung über Natriumsulfat und engt sie im Vakuum zur Trockne ein. Der Rückstand wird aus Aceton/Ether umkristallisiert und man erhält 3,19 g 4,5-Bis-(4-methoxyphenyl)-2-(4-pyridylthio)-imidazol, vom Schmelzpunkt 177-179 °C.

Beispiel 64

Zu einer Lösung von 3,90 g 4,5-Bis-(4-methoxyphenyl)-2-(4-pyridylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird aus Ethanol/Ether umkristallisiert und man erhält 3,41 g 4,5-Bis-(4-methoxyphenyl)-2-(4-pyridylsulfinyl)-imidazol vom Schmelzpunkt 170-171 °C.

$C_{24}H_{22}N_2O_3S$ (418,52)
Ber.: 68,88 % C 5,30 % H 6,69 % N 7,66 % S
Gef.: 68,75 % C 5,37 % H 6,60 % N 7,61 % S

Beispiel 65

Zu einer Lösung von 3,90 g 4,5-Bis-(4-methoxyphenyl)-2-(4-pyridylthio)-imidazol in 100 ml Dichlormethan tropft man die Lösung von 4,33 g 3-Chlorperbenzoesäure (80 %) in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,48 g 4,5-Bis-(4-methoxyphenyl)-2-(4-pyridylsulfonyl)-imidazol als amorphen Schaum.

$C_{22}H_{19}N_3O_5S$ (421,48)
Ber.: 62,69 % C 4,54 % H 9,97 % N 7,61 % S
Gef.: 62,58 % C 4,49 % H 9,90 % N 7,53 % S

Beispiel 66

Zu einer Lösung von 230 mg Natrium in 100 ml absolutem Ethanol gibt man 3,12 g 4,5-Bis-(4-methoxyphenyl)-2-mercaptoimidazol und nach Lösung 3,61 g Diphenyliodoniumbromid. Man erhitzt 2 Studen am Rückfluß, versetzt dann mit 115 mg Natrium sowie weiteren 1,81 g Diphenyliodoniumbromid und erhitzt nochmals 2 Stunden unter Rückfluß. Man läßt erkalten, gießt in 300 ml Eiswasser, nimmt das Produkt in Essigester auf, trocknet die organische Lösung über Natriumsulfat und engt sie im Vakuum ein. Das Jodbenzol wird durch Kodestillation mit Toluol entfernt. Der Rückstand wird aus Hexan/Essigester kristallisiert, und man erhält 3,34 g 4,5-Bis-(4-methoxyphenyl)-2-(phenylthio)-imidazol vom Schmelzpunkt 177 bis 179 °C.

Beispiel 67

Zu einer Lösung von 3,65 g 4,5-Bis-(4-methoxyphenyl)-1-(tetrahydropyran-2-yl)-imidazol in einem Gemisch aus 50 ml absolutem Ether und 50 ml Benzol werden unter Rühren und Argon als Schutzgas bei – 5° bis 0 °C 7 ml ca. 1,5 N n-Butyllithium in Hexan, das mit 10 ml Benzol verdünnt wurde, getropft. Nach 20 Minuten tropft man die Lösung von 2,65 g p-Toylsulfinsäure-p-toluthiolester in 30 ml absolutem Ether zu, wobei durch Kühlung die Temperatur zwischen – 5° und 0 °C gehalten wird. Nach 6 Stunden Rühren bei Raumtemperatur gießt man in 800 ml Wasser. Man nimmt das Produkt in Essigester auf, trocknet die organische Lösung über Natriumsulfat und engt sie zur Trockne ein. Der Rückstand wird an 250 g Aluminiumoxid, neutral, Aktivitätsstufe II, mit Essigester/Hexan 2 : 3 chromatographiert. Man erhält eine unpolare Fraktion, die aus Ether kristallisiert 1,58 g 4,5-Bis-(4-methoxyphenyl)-1-(tetrahydropyran-2-yl)-2-(4-methylphenylthio)-imidazol vom Schmelzpunkt 192 °C und eine polarere, die nach Kristallisation aus Ether/Hexan 1,09 g 4,5-Bis-(4-methoxyphenyl)-1-(tetrahydropyran-2-yl)-2-(4-methylphenylsulfinyl)-imidazol vom Schmelzpunkt 149-151 °C liefert. Die Abspaltung der Schutzgruppen wird wie in Beispiel 22 beschrieben vorgenommen, und man erhält aus der Thioverbindung 1,23 g 4,5-Bis-(4-methoxyphenyl)-2-(4-methylphenylthio)-imidazol, das nach Umkristallisation aus Ether bei 181 °C und aus der Sulfinylverbindung 0,83 g 4,5-Bis-(4-methoxyphenyl)-2-(4-methylphenylsulfinyl)-imidazol, das nach Umkristallisation aus Ethanol/Ether bei 162-164 °C schmilzt.

Herstellung des p-Tolyl-sulfinsäure-p-toluthiolesters: Zu einer Lösung von 4,93 g 4,4'-Dimethyldiphenyldisulfid in 50 ml Dichlormethan tropft man bei 0 °C unter Rühren die Lösung von 4,74 g (ca. 75 %) 3-Chlorperbenzoesäure in 500 ml Dichlormethan. Es bildet sich ein Niederschlag, der durch Zugabe von 30 ml absolutem Tetrahydrofuran in Lösung gebracht wird. Nach 5 Stunden wäscht man mit eiskalter Natriumbikarbonatlösung, trocknet über Natriumsulfat und engt im Vakuum zur Trockne ein. Der Rückstand wird in Ether aufgenommen und bei + 4 °C kristallisiert. Man erhält 3,12 g p-Tolylsulfinsäure-p-toluthiolester vom Schmelzpunkt 91 °C.

Beispiel 68

Zu einer Lösung von 4,85 g Pentafluorthiophenol in 35 ml Tetrachlorkohlenstoff gibt man 3 Tropfen Triethylamin und tropft dann bei 0° 2,07 ml Sulfurylchlorid in 35 ml Tetrachlorkohlenstoff zu. Man läßt 30 Minuten bei 0° nachrühren, zieht dann das Lösungsmittel im Vakuum ab und entfernt Reste Lösungsmittel durch Kodestillation mit absolutem Toluol. Man nimmt in 30 ml Toluol auf, dekantiert vom Triethylammoniumhydrochlorid und setzt das so gewonnene Pentafluorphenylsulfenylchlorid ohne weitere Reinigung in die nachfolgende Reaktionsstufe ein.

Zu einer Lösung von 8,83 g 4,5-Bis-(4-methoxyphenyl)-1-(tetrahydropyran-2-yl)-imidazol in einem Gemisch aus 75 ml absolutem Tetrahydrofuran und 75 ml Toluol werden unter Rühren und Argon als Schutzgas bei – 5° bis 0 °C 15 ml ca. 1,6 N n-Butyllithium in Hexan, das mit 30 ml Toluol verdünnt wurde, getropft. Nach 20 Minuten tropft man die Pentafluorphenylsulfenylchlorid-Lösung zu, wobei durch Kühlung die Temperatur zwischen – 5° und 0 °C gehalten wird. Nach 2 Stunden Rühren bei Raumtemperatur verdünnt man die Lösung mit 200 ml Essigester, wäscht mit Natrium-bikarbonatlösung, trocknet die organische Lösung über Natriumsulfat und engt im Vakuum zur Trockne ein. Der Rückstand wird durch Chromatographie an Aluminiumoxid, neutral, Aktivitätsstufe II mit Hexan/Essigester gereinigt. Nach Umkristallisation aus Essigester/Hexan erhält man 8,86 g 4,5-Bis-(4-methoxyphenyl)-1-(tetrahydropyran-2-yl)-2-(pentafluorphenylthio)-imidazol vom Schmelzpunkt 156-158 °C.

Man löst 5,63 g der Verbindung in 100 ml Ethanol, versetzt mit 5 ml konzentrierter Salzsäure in 50 ml Ethanol und erwärmt kurz auf dem Wasserbad, worauf sich ein Kristallbrei abzuscheiden beginnt. Man läßt erkalten, saugt die Kristalle ab und wäscht sie mit Alkohol und erhält so 5,35 g 4,5-Bis-(4-methoxyphenyl)-2-(pentafluorphenylthio)-imidazol Hydrochlorid vom Zersetzungspunkt 246-248 °C.

## Beispiel 69

Zu einer Lösung von 3,51 g 4,5-Bis-(4-methoxyphenyl)-1-(tetrahydrofuran-2-yl)-imidazol in einem Gemisch aus 80 ml absolutem Ether und 40 ml Toluol werden unter Argon und Rühren bei 0 °C 7 ml 1,5 N Butyllithium in Hexan, das mit 10 ml Toluol verdünnt wurde, getropft. Nach 20 Minuten tropft man die Lösung von 2,19 g Diphenyldisulfid in 15 ml absolutem Ether zu, wobei die Temperatur 20 °C nicht überschreitet. Nach 2 Stunden Rühren bei Raumtemperatur extrahiert man die Lösung mit 2 N Natronlauge. Die organische Lösung wird im Vakuum zur Trockne eingeengt.

Der Rückstand wird an 150 g Aluminiumoxid, neutral, Aktivitätsstufe II, chromatographiert. Mit Essigester/Hexan eluiert man 3,86 g 4,5-Bis-(4-methoxyphenyl)-1-(tetrahydrofuran-2-yl)-2-phenylthio-imidazol als amorphen Schaum.

$C_{27}H_{26}N_2O_3S$ (458,58)

| | | | | |
|---|---|---|---|---|
| Ber. : | 70,72 % C | 5,71 % H | 6,11 % N | 6,99 % S |
| Gef. : | 70,55 % C | 5,80 % H | 6,00 % N | 6,88 % S |

Die Verbindung wird in 50 ml Eisessig/Wasser gelöst und 10 Minuten auf dem Wasserbad erwärmt. Man zieht das Lösungsmittel im Vakuum ab, entfernt Reste Essigsäure durch Kodestillation mit Ethanol und kristallisiert den Rückstand aus Essigester/Hexan. Man erhält so 3,08 g 4,5-Bis-(4-methoxyphenyl)-2-phenylthio-imidazol vom Schmelzpunkt 177-179 °C.

Herstellung des 4,5-Bis-(4-methoxyphenyl)-1-tetrahydrofuran-2-yl)-imidazols :

In 150 ml absolutem 1,2-Dichlorethan werden 5,61 g 4,5-Bis-(4-methoxyphenyl)-imidazol suspendiert und mit 2,8 g 2,3-Dihydrofuran sowie 0,2 ml Zinntetrachlorid versetzt. Man erwärmt unter Rühren 12 Stunden auf 70°, läßt erkalten und gießt in 200 ml kalte Natriumbikarbonatlösung. Die organische Phase wird abgetrennt, über Natriumsulfat getrocknet und im Vakuum eingeengt. Der Rückstand wird in Dichlormethan gelöst und einer Hexanfällung unterworfen. Der Niederschlag wird einer Säulenchromatographie an 250 g Aluminiumoxid, neutral, Aktivitätsstufe II, unterworfen. Mit Essigester/Hexan 2 : 3 eluiert man 5,39 g 4,5-Bis-(4-methoxyphenyl)-1-(tetrahydrofuran-2-yl)-imidazol, das beim Abziehen des Lösungsmittels schaumig anfällt.

$C_{21}H_{22}N_2O_3$ (350,420)

| | | | |
|---|---|---|---|
| Ber. : | 71,98 % C | 6,33 % H | 7,99 % N |
| Gef. : | 72,03 % C | 6,40 % H | 7,91 % N |

## Beispiel 70

In 150 ml absolutem Dimethylformamid werden 6,25 g 4,5-Bis-(4-methoxyphenyl)-2-mercaptoimidazol gelöst und mit 0,60 g Natriumhydrid (80 % in Öl) versetzt. Nach beendeter Wasserstoffentwicklung kühlt man die Lösung auf 0 °C, versetzt mit einer Spatelspitze Kupferpulver und tropft eine auf 0 °C gekühlte Diazoniumsalzlösung, die aus 3,26 g 4-Trifluormethylanilin (99 %), 1,38 g Natriumnitrit sowie 10 ml 6 N Salzsäure bereitet wurde, zu. Man läßt 30 Minuten bei 0° rühren und dann auf Raumtemperatur kommen. Dann engt man die Lösung im Vakuum ein, verteilt den Rückstand zwischen Wasser und Essigester, trocknet die organische Lösung über Natriumsulfat und engt sie im Vakuum zur Trockne ein. Der Rückstand wird an 500 g Aluminiumoxid, neutral, Aktivitätsstufe II, mit Hexan/Essigester 3 : 2 als Elutionsmittel chromatographiert. Man eluiert 3,39 g 4,5-Bis-(4-methoxyphenyl)-2-(4-trifluormethyl-phenylthio)-imidazol, vom Schmelzpunkt 179-181 °C.

## Beispiel 71

Unter den Bedingungen des Beispiels 85 werden 6,25 g 4,5-Bis-(4-methoxyphenyl)-2-mercaptoimida-

zol in 150 ml Dimethylformamid mit 0,60 g Natriumhydrid (80 % in Öl) und einer Diazoniumsalzlösung, die aus 3,26 g 3-Trifluormethylanilin (99 %), 1,38 g Natriumnitrit sowie 10 ml 6 N Salzsäure bereitet wird, in Gegenwart einer Spatelspitze Kupferpulver umgesetzt. Man erhält nach Chromatographie gemäß Beispiel 85 3,27 g 4,5-Bis-(4-methoxyphenyl)-2-(3-trifluormethyl-phenylthio)-imidazol als amorphen Schaum.

$C_{23}H_{19}F_3N_2O_2S$

| | | | | | |
|---|---|---|---|---|---|
| Ber. : | 62,15 % C | 4,31 % H | 12,82 % F | 6,30 % N | 7,21 % S |
| Gef. : | 62,21 % C | 4,38 % H | 12,76 % F | 6,21 % N | 7,14 % S |

Beispiel 72

Zu einer Lösung von 4,57 g 4,5-Bis-(4-methoxyphenyl)-2-(4-trifluormethylphenylthio)-imidazol in 100 ml Dichlormethan tropft man die Lösung von 2,164 g 3-Chlorperbenzoesäure (80 %) in 150 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 3,78 g 4,5-Bis-(4-methoxyphenyl)-2-(4-trifluormethylphenylsulfinyl)-imidazol als amorphen Schaum.

$C_{24}H_{19}F_3N_2O_3S$ (472,49)

| | | | | | |
|---|---|---|---|---|---|
| Ber. : | 61,01 % C | 4,05 % H | 12,06 % F | 5,93 % N | 6,79 % S |
| Gef. : | 60,82 % C | 3,90 % H | 11,98 % F | 5,91 % N | 6,80 % S |

Beispiel 73

Zu einer Lösung von 4,57 g 4,5-Bis-(4-methoxyphenyl)-2-(3-trifluormethylphenylthio)-imidazol in 100 ml Dichlormethan tropft man die Lösung von 4,33 g 3-Chlorperbenzoesäure in 300 ml Dichlormethan. Man läßt 3 Stunden bei Raumtemperatur rühren, wäscht die Lösung mit Natriumbikarbonatlösung, trocknet sie über Natriumsulfat und engt sie im Vakuum zur Trockne ein.

Der Rückstand wird an 150 g Kieselgel mit Aceton/Hexan chromatographiert und man erhält 4,33 g 4,5-Bis-(4-methoxyphenyl)-2-(3-trifluormethylphenylsulfonyl)-imidazol als amorphen Schaum.

$C_{24}H_{19}F_3N_2O_4S$ (488,49)

| | | | | | |
|---|---|---|---|---|---|
| Ber. : | 59,01 % C | 3,92 % H | 11,67 % F | 5,73 % N | 6,56 % S |
| Gef. : | 59,12 % C | 4,03 % H | 11,68 % F | 5,73 % N | 6,54 % S |

Beispiel 74

Zu einer Lösung von 3,12 g 4,5-Bis-(4-methoxyphenyl)-2-mercaptoimidazol in 150 ml absolutem Dimethylformamid werden 0,6 g Natriumhydrid (80 % in Öl) gegeben. Nach beendeter Wasserstoffentwicklung läßt man 20 Minuten Nachrühren und versetzt unter Rühren anteilweise mit 1,76 g 2-Chlorpyridin-N-oxid Hydrochlorid. Man rührt 1 Stunde bei Raumtemperatur nach, zieht das Lösungsmittel im Vakuum ab, verdünnt mit wenig Ethanol, gießt in 200 ml Eiswasser und nimmt das ausgefallene Produkt in Dichlormethan auf. Man trocknet die organische Lösung über Natriumsulfat, engt sie im Vakuum zur Trockne ein und kristallisiert aus Dichlormethan/Hexan.

Man erhält so 3,06 g 4,5-Bis-(4-methoxyphenyl)-2-(N-oxi-2-pyridylthio)-imidazol vom Schmelzpunkt 215-217 °C.

Beispiel 75

Zu einer Lösung von 3,90 g 4,5-Bis-(4-methoxyphenyl)-2-(2-pyridylthio)-imidazol in 100 ml Dichlormethan gibt man 7,5 g 3-Chlorperbenzoesäure (80 %) in 600 ml Dichlormethan. Man läßt 7 Tage bei Raumtemperatur rühren, wäscht die Lösung mit Natriumbikarbonatlösung, trocknet sie mit Natriumsulfat, und engt sie im Vakuum zur Trockne ein. Der Rückstand wird an 150 g Kieselgel mit Aceton/Hexan 2 : 3 als Elutionsmittel chromatographiert. Man erhält so 2,88 g 4,5-Bis-(4-methoxyphenyl)-2-(N-oxi-2-pyridylsulfonyl)-imidazol, das nach Umkristallisation aus Aceton einen Zersetzungspunkt von 261-263 °C zeigt.

**Patentansprüche**

1. Imidazol-Derivate der allgemeinen Formel I

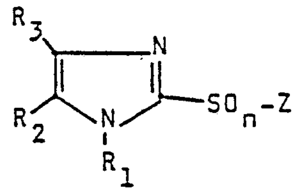

(I)

worin

$R_1$ ein Wasserstoffatom oder einen gegebenenfalls durch Methoxygruppen substituierter Alkylrest mit 1 bis 6 Kohlenstoffatomen bedeutet,

$R_2$ und $R_3$ einen gegebenenfalls durch Halogenatome, 1 bis 4 Kohlenstoffatome enthaltende Alkylreste oder 1 bis 4 Kohlenstoffatome enthaltende Alkoxyreste substituierten Phenylrest bedeuten,

n die Ziffern 0, 1 oder 2 darstellt und

Z einen gegebenenfalls durch Halogenatome, 1 bis 4 Kohlenstoffatome enthaltende Alkylgruppen, 1 bis 4 Kohlenstoffatome enthaltende Alkoxygruppen oder Trifluormethylgruppen substituierten Phenylrest, Pyridylrest, N-Oxipyridylrest, Pyrimidinylrest, Thiazolylrest oder Thienylrest bedeutet, und deren Salze mit physiologisch unbedenklichen Säuren.

2. Imidazol-Derivate der allgemeinen Formel I gemäß Anspruch 1, dadurch gekennzeichnet, daß die Reste $R_2$ und $R_3$ einen gegebenenfalls in para-Stellung durch ein Fluoratom, ein Chloratom, eine 1 bis 4 Kohlenstoffatome enthaltende Alkylgruppe oder eine 1 bis 4 Kohlenstoffatome enthaltende Alkoxygruppe substituierten Phenylrest darstellen.

3. Imidazol-Derivate der allgemeinen Formel I gemäß Anspruch 2, dadurch gekennzeichnet, daß $R_2$ und $R_3$ einen Phenylrest, einen 4-Fluorphenylrest, einen 4-Chlorphenylrest, einen 4-Methylphenylrest oder einen 4-Methoxyphenylrest darstellen.

4. Imidazol-Derivate der allgemeinen Formel I gemäß Anspruch 1 bis 3, dadurch gekennzeichnet, daß n die Ziffern 1 oder 2 bedeutet.

5. Imidazol-Derivate der allgemeinen Formel I gemäß Anspruch 1 bis 4, dadurch gekennzeichnet, daß der Substituent Z einen gegebenenfalls durch Fluoratome, Chloratome, Bromatome, Alkylgruppen mit 1 bis 4 Kohlenstoffatomen, Alkoxygruppen mit 1 bis 4 Kohlenstoffatomen oder Trifluormethylgruppen substituierter Phenylrest, einen 2-Pyridylrest, einen 4-Pyridylrest, einen N-Oxipyridylrest, einen 2-Pyrimidinylrest, einen Thienylrest oder einen Thiazolylrest darstellt.

6. 4,5-Bis-(4-methoxyphenyl)-2-(2-thienylthio)-imidazol.

7. 4,5-Bis-(4-methoxyphenyl)-2-phenylthio-imidazol.

8. 4,5-Bis-(4-methoxyphenyl)-2-(2-fluorphenylthio)-imidazol.

9. 4,5-Bis-(4-methoxyphenyl)-2-(4-fluorphenylthio)-imidazol.

10. 4,5-Bis-(4-methoxyphenyl)-2-(3-fluorphenylthio)-imidazol.

11. 4,5-Bis-(4-methoxyphenyl)-2-(3,4-dichlorphenylthio)-imidazol.

12. 4,5-Bis-(4-methoxyphenyl)-2-(4-chlorphenylthio)-imidazol.

13. 4,5-Bis-(4-methoxyphenyl)-2-phenylsulfonyl-imidazol.

14. Pharmazeutische Präparate, enthaltend ein oder zwei Imidazol-Derivate gemäß Anspruch 1 bis 13.

15. Pharmazeutische Präparate gemäß Anspruch 14 zur Behandlung entzündlicher oder allergischer Erkrankungen des Menschen.

16. Pharmazeutische Präparate gemäß Anspruch 14 zur Behandlung von Migräne.

17. Pharmazeutische Präparate gemäß Anspruch 14 zur Behandlung von Dysmenorrhoe.

18. Verfahren zur Herstellung von Imidazol-Derivaten der allgemeinen Formel I gemäß Patentanspruch 1 und deren Salze mit physiologisch unbedenklichen Säuren, dadurch gekennzeichnet, daß man

a) eine Verbindung der allgemeinen Formel II

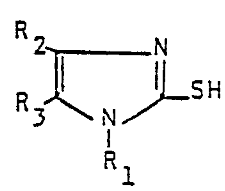

(II)

worin $R_1$, $R_2$ und $R_3$ die obengenannte Bedeutung besitzen, in Gegenwart von Basen mit einer Verbindung der allgemeinen Formel III

XZ (III)

worin

Z die obengenannte Bedeutung besitzt und

X ein Halogenatom, einen Alkylsulfonylrest, einen Arylsulfonylrest oder eine Diazogruppe darstellt, kondensiert, oder

b) eine Verbindung der allgemeinen Formel IV

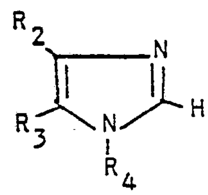

(IV)

worin

$R_2$ und $R_3$ die obengenannte Bedeutung besitzen und

$R_4$ die gleiche Bedeutung wie $R_1$ hat, aber kein Wasserstoff atom darstellt,

in Gegenwart von Basen mit einer Verbindung der allgemeinen Formel V, VI, VII, VIII oder IX

$$Cl{-}S{-}Z \qquad (V)$$
$$Z{-}S{-}S{-}Z \qquad (VI)$$
$$R_3{-}O{-}SO{-}Z \qquad (VII)$$
$$Z{-}S{-}SO{-}Z \qquad (VIII)$$
$$Z{-}SO_2{-}O{-}SO_2{-}Z \qquad (IX)$$

worin Z die obengenannte Bedeutung besitzt und $R_3$ einen Alkylrest von 1 bis 4 Kohlenstoffatomen darstellt, kondensiert

und gegebenenfalls die gemäß Verfahrensvariante a) oder b) hergestellte Verbindung oxidiert, hydrolisiert, alkyliert, und/oder diese Verbindungen mit physiologisch unbedenklichen Säuren in ihre Salze überführt.

**Claims**

1. Imidazole derivates of general formula I

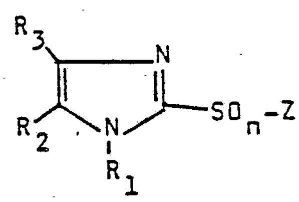

(I)

wherein

$R^1$ is hydrogen or an alkyl group of 1 to 6 carbon atoms, optionally substituted by a methoxy group, $R^2$ and $R^3$ is a phenyl group, optionally substituted by halogen, alkyl of 1 to 4 carbon atoms or alkoxy of 1 to 4 carbon atoms,

n is the integer 0, 1 or 2, and

Z is a phenyl group, optionally substituted by halogen, alkyl of 1 to 4 carbon atoms, alkoxy of 1 to 4 carbon atoms or trifluoromethyl, pyridyl,

N-oxypyridyl, pyrimidinyl, thiazolyl or thienyl, and salts of these with physiologically acceptable acids.

2. Imidazole derivatives of general formula I according to claim 1, characterised in that the groups $R_2$ and $R_3$ are phenyl optionally substituted in the para position by fluorine, chlorine, alkyl of 1 to 4 carbon atoms or alkoxy of 1 to 4 carbon atoms.

3. Imidazole derivatives of general formula I according to claim 2 characterised in that $R_2$ and $R_3$ are 4-fluorophenyl, 4-chlorophenyl, 4-methylphenyl or 4-methoxyphenyl.

4. Imidazole derivatives of general formula I according to any of claims 1 to 3 characterised in that n is the integer 1 or 2.

5. Imidazole derivatives of general formula I according to any of claims 1 to 4 characterised in that the substituent Z is phenyl optionally substituted by fluorine, chlorine, bromine, alkyl of 1 to 4 carbon atoms, alkoxy of 1 to 4 carbon atoms or trifluoromethyl, 2-pyridyl, 4-pyridyl, N-oxypyridyl, 2-pyrimidinyl, thienyl or thiazolyl.

6. 4,5-Bis-(4-methoxyphenyl)-2-(2-thienylthio) imidazole.

7. 4,5-Bis-(4-methoxyphenyl)-2-phenylthioimidazole.

8. 4,5-Bis-(4-methoxyphenyl)-2-(2-fluorophenylthio)-imidazole.
9. 4,5-Bis-(4-methoxyphenyl)-2-(4-fluorophenylthio)-imidazole.
10. 4,5-Bis-(4-methoxyphenyl)-2-(3-fluorophenylthio)-imidazole.
11. 4,5-Bis-(4-methoxyphenyl)-2-(3,4-dichlorophenylthio)-imidazole.
12. 4,5-Bis-(4-methoxyphenyl)-2-(4-chlorophenylthio)-imidazole.
13. 4,5-Bis-(4-methoxyphenyl)-2-phenylsulphonyl)-imidazole.
14. Pharmaceutical compositions containing one or two imidazole derivatives according to claims 1 to 13.
15. Pharmaceutical compositions according to claim 14 for the treatment of inflammatory or allergic illnesses in humans.
16. Pharmaceutical compositions according to claim 14 for the treatment of migraine.
17. Pharmaceutical compositions according to claim 14 for the treatment of dysmenorrhoea.
18. Process for the preparation of imidazole derivatives of general formula I according to claim 1 and their salts with physiologically acceptable acids, characterised in that

a) a compound of general formula II

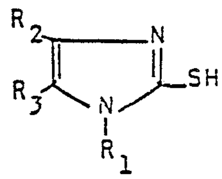 ; (II)

in which $R_1$, $R_2$ and $R_3$ have the meanings given above, is condensed, in the presence of a base with a compound of general formula III

$$XZ \qquad (III)$$

in which Z has the meaning given above and X is halogen, alkylsulphonyl, arysulphonyl or a diazo group, or

b) a compound of general formula IV

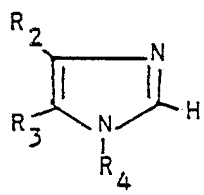 (IV)

wherein $R_2$ and $R_3$ have the meaning given above and $R_4$ has the same meaning as $R_1$, except hydrogen, is condensed, in the presence of a base with a compound of general formula V, VI, VII, VIII or IX

$$Cl—S—Z \qquad (V)$$
$$Z—S—S—Z \qquad (VI)$$
$$R_3—O—SO—Z \qquad (VII)$$
$$Z—S—SO—Z \qquad (VIII)$$
$$Z—SO_2—O—SO_2—Z \qquad (IX)$$

wherein Z has the meaning given above and $R_3$ is alkyl of 1 to 3 carbon atoms and optionally, the compound obtained in process variants (a) or (b) is oxidised and/or these compounds are converted into their salts with physiologically acceptable acids.

**Revendications**

1. Dérivés de l'imidazole répondant à la formule générale I :

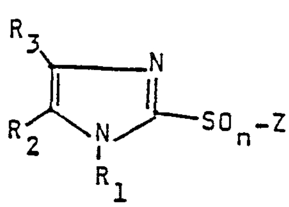 (I)

dans laquelle

$R_1$ représente un atome d'hydrogène ou un radical alkyle alkyle qui contient de 1 à 6 atomes de carbone et qui porte éventuellement des radicaux méthoxy,

$R_2$ et $R_3$ représentent chacun un radical phényle qui porte éventuellement des atomes d'halogènes, des radicaux alkyles contenant de 1 à 4 atomes de carbone, ou des radicaux alcoxy contenant de 1 à 4 atomes de carbone, ou des radicaux alcoxy contenant de 1 à 4 atomes de carbone,

n représente le nombre 0, le nombre 1 ou le nombre 2 et

Z représente un radical phényle éventuellement porteur d'atomes d'halogènes, de radicaux alkyles contenant de 1 à 4 atomes de carbone, de radicaux alcoxy contenant de 1 à 4 atomes de carbone ou de radicaux trifluoro-méthyles, un radical pyridyle, un radical N-oxy-pyridyle, un radical pyrimidinyle, un radical thiazolyle ou un radical thiényle,

et ses sels formés par ces composés avec des acides acceptables du point de vue physiologique.

2. Dérivés de l'imidazole de formule générale I selon la revendication 1, caractérisés en ce que les symboles $R_2$ et $R_3$ représentent chacun un radical phényle qui porte éventuellement, en position para, un atome de fluor ou de chlore, un radical alkyle contenant de 1 à 4 atomes de carbone ou un radical alcoxy contenant de 1 à 4 atomes de carbone.

3. Dérivés de l'imidazole de formule générale I selon la revendication 2, caractérisés en ce que $R_2$ et $R_3$ représentent chacun un radical phényle, un radical fluoro-4 phényle, un radical chloro-4 phényle, un radical méthyl-4 phényle ou un radical méthoxy-4 phényle.

4. Dérivés de l'imidazole de formule générale I selon l'une quelconque des revendications 1 à 3, caractérisés en ce que n représente le nombre 1 ou le nombre 2.

5. Dérivés de l'imidazole de formule générale I selon l'une quelconque des revendications 1 à 4, caractérisés en ce que le symbole Z représente un radical phényle qui porte éventuellement des atomes de fluor, des atomes de chlore, des atomes de brome, des radicaux alkyles contenant de 1 à 4 atomes de carbone, des radicaux alcoxy contenant de 1 à 4 atomes de carbone, ou des radicaux trifluorométhyles, un radical pyridyle-2, un radical pyridyle-4, un radical N-oxy pyridyle, un radical pyrimidinyle-2, un radical thiényle ou un radical thiazolyle.

6. Bis-(méthoxy-4 phényl)-4,5 (thiényl-2 thio)-2 imidazole.

7. Bis-(méthoxy-4 phényl)-4,5 phénylthio-2 imidazole.

8. Bis-(méthoxy-4 phényl)-4,5 (fluoro-2 phénylthio)-2 imidazole.

9. Bis-(méthoxy-4 phényl)-4,5 (fluoro-4 phénylthio)-2 imidazole.

10. Bis-(méthoxy-4 phényl)-4,5 (fluoro-3 phénylthio)-2 imidazole.

11. Bis-(méthoxy-4 phényl)-4,5 (dichloro-3,4 phénylthio)-2 imidazole.

12. Bis-(méthoxy-4 phényl)-4,5 (chloro-4 phénylthio)-2 imidazole.

13. Bis-(méthoxy-4 phényl)-4,5 phénylsulfonyl-2 imidazole.

14. Médicaments contenant un ou deux dérivés de l'imidazole selon l'une quelconque des revendications 1 à 13.

15. Médicaments selon la revendication 14 pour le traitement de maladies inflammatoires ou allergiques de l'homme.

16. Médicaments selon la revendication 14 pour le traitement de migraines.

17. Médicaments selon la revendication 14 pour le traitement dysménorrhées.

18. Procédé de préparation de dérivés de l'imidazole de formule générale I selon la revendication 1, et de leurs sels formés avec des acides acceptables du point de vue physiologique, procédé caractérisé en ce que :

a) on condense un composé répondant à la formule générale II :

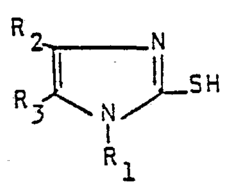

(II)

dans laquelle $R_1$, $R_2$ et $R_3$ ont les significations précédemment données, en présence de bases, avec un composé répondant à la formule générale III :

$$XZ \qquad (III)$$

dans laquelle Z a la signification précédemment donnée et X représente un atome d'halogène, un radical alcane-sulfonyle, un radical arène-sulfonyle ou un radical diazo,

b) on condense un composé répondant à la formule générale IV :

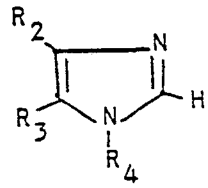

(IV)

dans laquelle $R_2$ et $R_3$ ont les significations précédemment données et $R_4$ a l'une des significations qui ont été données ci-dessus pour $R_1$ mais ne peut pas représenter un atome d'hydrogène, en présence de bases, avec un composé répondant à l'une des formules générales V, VI, VII, VIII et IX :

$$Cl—S—Z \quad (V)$$
$$Z—S—S—Z \quad (VI)$$
$$R_3—O—SO—Z \quad (VII)$$
$$Z—S—SO—Z \quad (VIII)$$
$$Z—SO_2—O—SO_2—Z \quad (IX)$$

dans lesquelles Z a la signification précédemment donnée et $R_3$ représente un radical alkyle contenant de 1 à 4 atomes de carbone,
et éventuellement on oxyde, on hydrolyse, on alkyle le composé préparé selon la variante a) ou la variante b), et/ou on transforme le composé obtenu en un sel avec un acide acceptable du point de vue physiologique.